# EUROPEAN PATENT APPLICATION

(11) **EP 0 934 934 A2**
(43) Date of publication of application: **11.08.1999**
(21) Application number: 99300839.0
(22) Date of filing: 04.02.1999
(51) Int. Cl.: C07D 239/91, C07D 401/06, C07D 401/04

(54) **Methods of preparing substituted 3-phenyl- and 3-pyridyl-4(3H)-quinazolinones and atropisomers thereof**

(30) Priority: 09.02.1998 US 74150 P
(71) Applicant: Pfizer Products Inc., Groton, Connecticut 06340 (US)
(72) Inventor: Chenard, Bertrand Leo, Waterford, Connecticut 06385 (US); Shenk, Kevin Dale, Groton, Connecticut 06340 (US)
(74) Representative: McMunn, Watson Palmer

(57) **Abstract**

This invention is directed to methods for the preparation of quinazolin-4-one derivatives of the formula 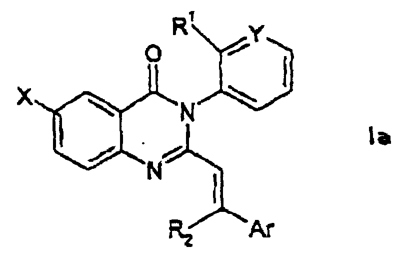 and the pharmaceutically acceptable salts and atropisomers of the formula 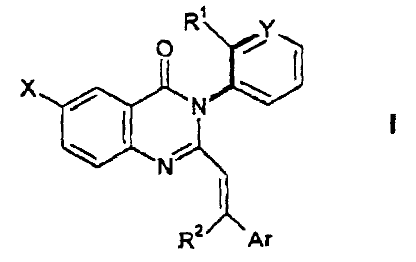 and the pharmaceutically acceptable salts thereof, wherein Ar, R₁, R₂, X and Y are as defined in the specification. The quinazolin-4-one derivatives prepared according to the methods of this invention are α-amino-3-hydroxy-5-methyl-4-isoxazole propionic acid (AMPA) inhibitors and are useful for the treatment of various neurologic disorders and conditions including Parkinson's Disease, epilepsy, emesis, eschemia, stroke, traumatic brain and spinal cord injury, and so forth.

The present invention also relates to compounds of the formulae

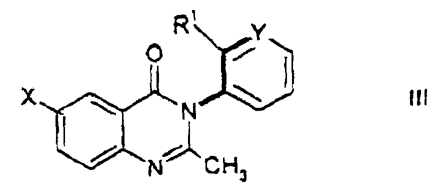

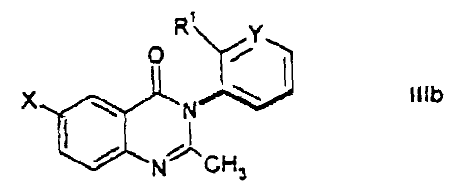

## Description

### Background of The Invention

This invention relates to a method for the preparation of certain quinazolin-4-one derivatives and the pharmaceutically acceptable salts thereof, which are potent AMPA (α-amino-3-hydroxy-5-methyl-4-isoxazole propionic acid) receptor antagonists useful in the treatment of various neurological disorders and conditions including Parkinson's Disease, epilepsy, emesis, ischemia, stroke, traumatic brain and spinal cord injury, and the like.

The role of excitatory amino acids (EAA), such as glutamic and aspartic acid, as the predominant mediators of excitatory synaptic transmission in the central nervous system has been well established. See, for example, Watkins & Evans, Ann. Rev. Pharmacol. Toxicol., 21, 165 (1981); Monaghan, Bridges and Cotman, Ann. Rev. Pharmacol. Toxicol., 29, 365 (1989); Watkins, Krogsgaard-Larsen and Honore, Trans. Pharm. Sci., 11, 25 (1990). These amino acids function in synaptic transmission primarily through excitatory amino acid receptors and participate in a variety of physiological processes including motor control, respiration, cardiovascular regulation, sensory perception and cognition.

Excitatory amino acid receptors may be classified into two general categories. Receptors that are coupled directly to the opening of cation channels in the cell membrane of the neuron are termed "ionotropic". The ionotropic receptors may be divided further into at least three subtypes, which are defined by the depolarizing actions of the selective agonists N-methyl-D-aspartate (NMDA), α-amino-3-hydroxy-5-methyl-4-isoxazole propionic acid (AMPA), and kainic acid (KA). The second general type of excitatory amino acid receptor is known as the G-Protein or second messenger-linked "metabotropic" excitatory amino acid receptor. This second type, when activated by the agonists quisqualate, ibotenate, or trans-1-aminocyclopentane-1,3-dicarboxylic acid, leads to enhanced phosphoinosoitide hydrolysis in the post-synaptic cell. Both types of receptors appear to not only mediate normal synaptic transmission along excitatory pathways, but also participate in the modification of synaptic connection during development and changes in the efficiency of synaptic transmission throughout life. See Schoepp, Bockaert and Sladeczek, Trends in Pharmacol. Sci., 11, 508 (1990); McDonald and Johnson, Brain Research Reviews, 15, 41(1990).

The excessive or inappropriate stimulation of excitatory amino acid receptors leads to neuronal cell degeneration or death by way of a mechanism known as excitotoxicity and it is believed that this cellular degeneration is mediated in part by the excitotoxic actions of the excitatory amino acids glutamate and aspartate at the NMDA, AMPA and kainate receptors. The medical consequences of such neuronal degradation make the abatement of these degenerative neurological processes an important therapeutic objective.

EAA excitotoxicity has been implicated in the pathophysiology of a number of acute and chronic neurological disorders and neurodegenerative conditions including cerebral deficits subsequent to cardiac bypass surgery and grafting, stroke, cerebral ischemia, head and spinal cord trauma, Alzheimer's Disease, Huntington's Chorea, amyotrophic laterosclerosis, epilespsy, AIDS-induced dementia, perinatal hypoxia, cardiac arrest, hypoglycemic neuronal damage, ocular damage, and retinopathy, and idopathic and drug-induced Parkinson's Disease. Other neurological conditions that result from glutamate dysfunction and which require neuromodulation include muscular spasms, migrane headaches, urinary incontinence, psychosis, addiction withdrawal (such as alcoholism and drug addiction including opiate, cocaine and nicotine addiction), opiate tolerance, anxiety, emesis, brain edema, chronic pain, convulsions, retinal neuropathy, tinnitus, and tardive dyskinesia. The use of a neuroprotective agent, such as an AMPA receptor antagonist, is believed to be useful in treating these disorders and/or attenuating the amount of neurological damage associated with these conditions. The EAA antagonists are also useful as analgesic agents. See Xu, Hao, Seiger and Wisenfeld-Hallin; J. Pharmacol. Exp. Ther. 267, 140 (1993).

Several studies have shown that AMPA receptor antagonists are neuroprotective in both focal and global models of ischemia. The competitive AMPA receptor antagonist NBQX (2,3-dihydroxy-6-nitro-7-sulfamoylbenzo[f]quinoxaline) has been reported to be effective in the prevention damage due to both of focal and global ischemia. Sheardown et al., Science, 247, 571 (1990); Buchan et al., Neuroreport, 2, 473 (1991); LePeillet et al., Brain Research, 571, 115 (1992). The non-competitive AMPA receptor antagonist GKYI 52466 has been shown to be an effective neuroprotective agent in rat global ischemia models (LePeillet et al., Brain Research, 571, 115 (1992)). These studies suggest strongly that the delayed neuronal degeneration in brain ischemia involves glutamate excitotoxicity mediated, at least in part, by AMPA receptor activation. Thus, AMPA receptor antagonists may prove useful as neuroprotective agents and improve the neurological outcome of cerebral ischemia in humans.

### Summary of the Invention

The present invention relates to a process for preparing a compound of the formula 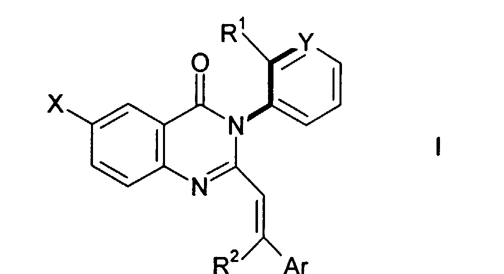 and the pharmaceutically acceptable salts thereof, wherein
R¹ is halo, cyano, (C₁-C₆)alkyl, perfluoro(C₁-C₆)alkyl or -COO(C₁-C₆)alkyl;
R² is hydrogen or hydroxy;
X is hydrogen, hydroxy, halo, trifluoromethyl, nitro, (C₁-C₆)alkyl optionally substituted by (C₁-C₆)alkoxy, hydroxy, amino (C₁-C₆)alkylamino or ((C₁-C₆)alkyl)₂amino; (C₃-C₇)cycloalkyl, (C₁-C₆)alkoxy, (C₁-C₆)acyl, (C₁-C₆)alkyl-CO₂-(C₁-C₆)alkyl, (C₁-C₆)alkyl-OCO-(C₁-C₆)alkyl, (C₁-C₆)alkyl-CO₂-, NCO, R³OCO-, R³NHCO- wherein R³ is hydrogen or (C₁-C₆)alkyl; ((C₁-C₆)alkyl)amino-CO- or phenyl optionally substituted by halo, (C₁-C₆)alkyl, cyano or trifluoromethyl;
Y is nitrogen or CH;
Ar is a group of the formula 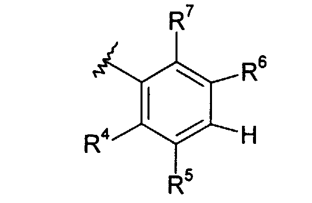 wherein R⁴ and R⁷ are each independently selected from hydrogen, halo, trifluoromethyl, (C₁-C₆)alkoxy optionally substituted with one to three halogen atoms; (C₁-C₆)alkylthio, hydroxy, (C₁-C₆)acyl, cyano, (C₁-C₆)alkyl optionally substituted by amino, (C₁-C₆)alkylamino, ((C₁-C₆)alkyl)₂amino, (C₃-C₇)cycloalkylamino, hydroxy, (C₁-C₆)alkoxy, HCO- H₂NCO-, (C₁-C₆)alkylamino-CO-, ((C₁-C₆)alkyl)₂amino-CO-, (C₃-C₇)cycloalkylamino-CO-, HCO-NH-, (C₁-C₆)alkyl-CO-NH-, HCO-N((C₁-C₆)alkyl)-, (C₁-C₆)alkyl-CO-N((C₁-C₆)alkyl)-, one to three halogen atoms, R⁸O- or R⁸O-CO- wherein R⁸ is hydrogen, (C₁-C₆)alkyl, (C₁-C₆)acyl, (C₁-C₆)alkoxy-CO-, (C₁-C₆)alkylamino-CO-, or ((C₁-C₆)alkyl)₂amino-CO-;
R⁵ and R⁶ are each independently selected from hydrogen, halo, CF₃, (C₁-C₆)alkyl optionally substituted with one to three halogen atoms, (C₁-C₆)alkoxy optionally substituted with one to three halogen atoms, (C₁-C₆)alkylthiol, amino-(CH₂)ₚ-, (C₁-C₆)alkyl-NH-(CH₂)ₚ-, di(C₁-C₆)alkyl-N-(CH₂)ₚ-, (C₃-C₇)cycloalkyl-NH-(CH₂)ₚ-, amino-(C₁-C₆)alkyl-NH-(CH₂)ₚ-, (C₁-C₆)alkyl-NH-(C₁-C₆)alkyl-NH-(CH₂)ₚ-, di(C₁-C₆)alkyl-N((C₁-C₆)alkyl)-(C₁-C₆)alkyl-NH(CH₂)ₚ-, di(C₁-C₆)alkyl-N-(C₁-C₆)alkyl-N-(CH₂)ₚ-, H₂N-(C=O)-(CH₂)ₚ-, (C₁-C₆)alkyl-HN-(C=O)-(CH₂)ₚ-, di(C₁-C₆)alkyl-N-(C=O)-(CH₂)ₚ, (C₃-C₇)cycloalkyl-NH-(C=O)-(CH₂)ₚ-, R¹³O-(CH₂)ₚ-, R¹³O-(C=O)-(CH₂)ₚ-, H(O=C)-O-, H(O=C)-O-(C₁-C₆)alkyl-, H(O=C)-NH-(CH₂)ₚ-, (C₁-C₆)alkyl-(O=C)-NH-(CH₂)ₚ-, -CHO, H-(C=O)-(CH₂)ₚ-, (C₁-C₆)alkyl-(C=O)-(CH₂)ₚ-, (C₁-C₆)alkyl-(O=C)-N((C₁-C₆)alkyl)-(CH₂)ₚ-, H(O=C)-N((C₁-C₆)alkyl)-(CH₂)ₚ-, HO-(C₁-C₆)alkyl-N((C₁-C₆)alkyl)-(CH₂)ₚ-, (C₁-C₆)alkyl-(C=O)-O-NH-(CH₂)ₚ-, amino-(C₁-C₆)alkyl-(C=O)-O-(CH₂)ₚ-, (C₁-C₆)alkyl-NH-(C₁-C₆)alkyl-(C=O)-O-(CH₂)ₚ-, di(C₁-C₆)alkyl-N-(C₁-C₆)alkyl-(C=O)-O-(CH₂)ₚ-, amino-(C₁-C₆)alkyl-O-(C=O)-(CH₂)ₚ-, (C₁-C₆)alkyl-NH-(C₁-C₆)alkyl-O-(C=O)-(CH₂)ₚ-, di(C₁-C₆)alkyl-N-(C₁-C₆)alkyl-O-(C=O)-(CH₂)ₚ-, hydroxy, hydroxy-(C₁-C₆)alkyl-, hydroxy-(C₁-C₆)alkyl-NH-(CH₂)ₚ-, (C₁-C₆)alkyl-O-(C₁-C₆)alkyl-, -CN, piperidine-(CH₂)ₚ-, pyrrolidine-(CH₂)ₚ-, and 3-pyrroline-(CH₂)ₚ-, wherein said piperidine, pyrrolidine and 3-pyrroline of said piperidine-(CH₂)ₚ, pyrrolidine-(CH₂)ₚ- and 3-pyrroline-(CH₂)ₚ- moieties may optionally be substituted on any of the ring carbon atoms capable of supporting and additional bond, with a substituent independently selected from halo, CF₃, (C₁-C₆)alkyl optionally substituted with one to three halogen atoms, (C₁-C₆)alkoxy optionally substituted with one to three halogen atoms, (C₁-C₆)alkylthiol, amino-(CH₂)ₚ-, (C₁-C₆)alkyl-NH-(CH₂)ₚ-, di(C₁-C₆)alkyl-N-(CH₂)ₚ-, (C₃-C₇)cycloalkyl-NH-(CH₂)ₚ-, amino-(C₁-C₆)alkyl-NH-(CH₂)ₚ-, (C₁-C₆)alkyl-NH-(C₁-C₆)alkyl-NH-(CH₂)ₚ-, di(C₁-C₆)alkyl-N-C₁-C₆)alkyl-NH-(CH₂)ₚ-, (C₁-C₆)alkyl-O-(C₁-C₆)alkyl-, di(C₁-C₆)alkyl-N-(C₁-C₆)alkyl-N((C₁-C₆)alkyl)-(CH₂)ₚ-, H₂N-(C=O)-(CH₂)ₚ-, (C₁-C₆)alkyl-HN-(C=O)-(CH₂)ₚ-, di(C₁-C₆)alkyl-N-(C=O)-(CH₂)ₚ, (C₃-C₇)cycloalkyl-NH-(C=O)-(CH₂)ₚ-, R¹³O-(CH₂)ₚ-, R¹³O-(C=O)-(CH₂)ₚ-, H(O=C)-O-, H(O=C)-O-(C₁-C₆)alkyl-, H(O=C)-NH-(CH₂)ₚ-, (C₁-C₆)alkyl-(O=C)-NH-(CH₂)ₚ-, -CHO, H-(C=O)-(CH₂)ₚ-, (C₁-C₆)alkyl-(C=O)-, (C₁-C₆)alkyl-(O=C)-N((C₁-C₆)alkyl)-(CH₂)ₚ-, H(O=C)-N((C₁-C₆)alkyl)-(CH₂)ₚ-, HO-(C₁-C₆)alkyl-N((C₁-C₆)alkyl)-(CH₂)ₚ-, (C₁-C₆)alkyl- (C=O)-O-NH-(CH₂)ₚ-, amino-(C₁-C₆)alkyl-(C=O)-O-(CH₂)ₚ-, (C₁-C₆)alkyl-NH-(C₁-C₆)alkyl-(C=O)-O-(CH₂)ₚ-, di(C₁-C₆)alkyl-N-(C₁-C₆)alkyl-(C=O)-O-(CH₂)ₚ-, hydroxy, hydroxy-(C₁-C₆)alkyl-, hydroxy-(C₁-C₆)alkyl-NH-(CH₂)ₚ-, and -CN;
p is 0 to 6;
or Ar is a group of the formula 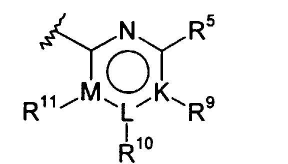 wherein K, L and M are each independently selected from carbon or nitrogen;
R⁵ is defined as above;
R⁹ and R¹⁰ are each independently selected from hydrogen, cyano, (C₁-C₆)alkyl, halo, trifluoromethyl, HCO- or (C₁-C₆)alkoxy;
R¹¹ hydrogen, cyano, halo, trifluoromethyl, (C₁-C₆)alkoxy, HCO-, (C₁-C₆)alkyl optionally substituted by amino, (C₁-C₆)alkylamino or ((C₁-C₆)alkyl)₂amino;
or Ar is a group of the formula 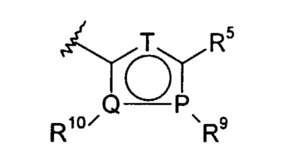 wherein T is CH, N, NH, O or S;
P and Q are each independently selected from carbon, nitrogen, oxygen or sulfur;
R⁵, R⁹ and R¹⁰ are defined as above;
with the proviso that only one of K, L or M can be nitrogen and when K, L or M is nitrogen then its respective R⁹, R¹⁰ or R¹¹ is absent; and
with the proviso that only one of P, Q or T can be oxygen or sulfur and at least one of P, Q or T must be a heteroatom;
comprising either (a) dehydrating the quinazolin-4-one derivative of the formula 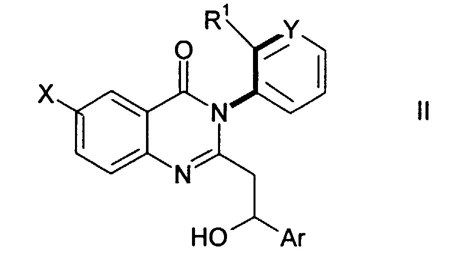 with a dehydration agent to form the compound of formula **I**, wherein R¹, X, Y and Ar are defined as above and R² is hydrogen; or
(b) oxidizing the compound of formula **II** with a oxidizing agent to form the compound of formula I, wherein R¹, X, Y and Ar are defined as above and R² is hydroxy.

The term "alkyl", as used herein, unless otherwise indicated, includes saturated monovalent hydrocarbon radicals having straight, branched or cyclic moieties or combinations thereof.

The term "alkoxy", as used herein, includes O-alkyl groups wherein "alkyl" is defined above.

The term "acyl", as used herein, unless otherwise indicated, includes a radical of the general formula RCO wherein R is alkyl, alkoxy, aryl, arylalkyl or arylalkyloxy and the terms "alkyl" or "aryl" are as defined above.

The phrase "pharmaceutically acceptable salt(s)", as used herein, unless otherwise indicated, includes salts of acidic or basic groups which may be present in the compounds of the present invention. The compounds of the present invention that are basic in nature are capable of forming a wide variety of salts with various inorganic and organic acids. The acids that may be used to prepare pharmaceutically acceptable acid addition salts of such basic compounds of are those that form non-toxic acid addition salts, i.e., salts containing pharmacologically acceptable anions, such as the hydrochloride, hydrobromide, hydroiodide, nitrate, sulfate, bisulfate, phosphate, acid phosphate, isonicotinate, acetate, lactate, salicylate, citrate, acid citrate, tartrate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, p-toluenesulfonate and pamoate [i.e., 1,1'-methylene-bis-(2-hydroxy-3-naphthoate)] salts. The compounds of the present invention that include an amino moiety may form pharmaceutically acceptable salts with various amino acids, in addition to the acids mentioned above.

Those compounds of the present invention that are acidic in nature are capable of forming base salts with various pharmacologically acceptable cations. Examples of such salts include the alkali metal or alkaline earth metal salts and, particularly, the calcium, magnesium, sodium and potassium salts of the compounds of the present invention.

It will be recognized that compounds of formula **Ia, IIa** or **IIIa**, the respective racemate compounds, exist as a separable pair of atropisomers. Atropisomers are isomeric compounds that are chiral, i.e., each isomer is not superimposable on its mirror image and the isomers, once separated, rotate polarized light in equal but opposite directions. Atropisomers are distinguished from enantiomers in that atropisomers do not possess a single asymmetric atom. Such compounds are conformational isomers which occur when rotation about a single bond in the molecule is prevented or greatly slowed as a result of steric interactions with other parts of the molecule and the substituents at both ends of the single bond are unsymmetrical. A detailed account of atropisomers can be found in Jerry March, Advanced Organic Chemistry, 101-102 (4th ed. 1992) and in Oki, Top. Stereochem., 14, 1-81 (1983).

The racemic compound of formula la 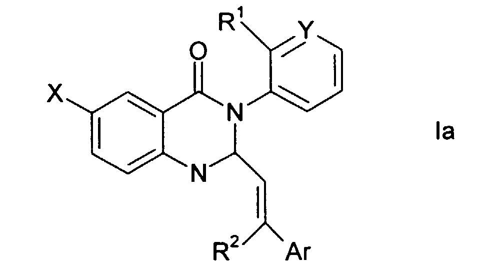 can exist as a separable pair of atropisomers as the compounds of formula I and Ib: 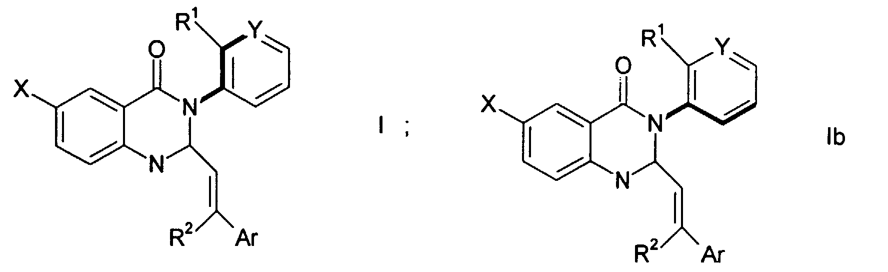 The bold lines in formulas **I** and **Ib** indicate that the bolded atoms, and the groups attached thereto, are sterically restricted so as to exist orthogonally above the plane of the quinazolin-4-one ring. The steric restriction is due to a rotational energy barrier preventing free rotation about the single bond connecting the nitrogen at position "3" of the quinazolin-4-one ring to the Y-containing (i.e., phenyl or pyridyl) aryl group.

A process for preparing the compound of formula **I**, wherein the dehydration agent is selected from acetic anhydride, trifluoroacetic anhydride, acetyl chloride, methanesulfonyl chloride, Burgess salt, thienyl chloride, phosphorus triichloride or phosphorus oxychloride.

A process for preparing the compound of formula **I**, wherein the oxidizing agent is selected from chromium trioxide, pyridinium dichromate, pyridinium chlorochromate, dicyclohexylcarbodiimide or ethyl 2-dimethylaminoethyl carbodiimide.

The present invention also relates to a process wherein the compound of the formula 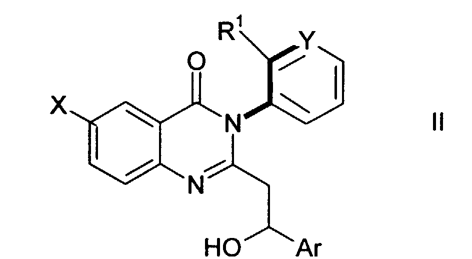 is formed by (a) deprotonating the methyl group at the 2-position of a quinazolin-4-one compound having the formula 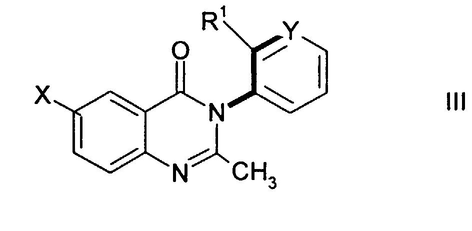 wherein X, Y and R¹ are as defined above, with a base to form the corresponding anion; and (b) reacting the anionic intermediate so formed with an aromatic aldehyde of the formula Ar-CHO, wherein Ar is defined as above, to form the compound of the formula **II**.

A process for preparing the compound of formula **II**, wherein the base is selected from lithium diisopropylamide, lithium hexamethyldisilylamide, sodium hexamethyldisilylamide, potassium hexamethyldisilylamide or lithium 2,2,6,6-tetramethylpiperidide.

The present invention also relates to a process for preparing a comDound of the formula 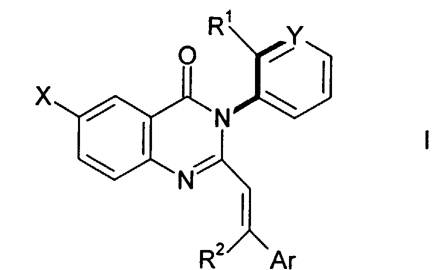 and the pharmaceutically acceptable salts thereof, wherein
R¹ is halo, cyano, (C₁-C₆)alkyl, perfluoro(C₁-C₆)alkyl or -COO(C₁-C₆)alkyl;
R² is hydrogen or hydroxy;
X is hydrogen, hydroxy, halo, trifluoromethyl, nitro, (C₁-C₆)alkyl optionally substituted by (C₁-C₆)alkoxy, hydroxy, amino (C₁-C₆)alkylamino or ((C₁-C₆)alkyl)₂amino; (C₃-C₇)cycloalkyl, (C₁-C₆)alkoxy, (C₁-C₆)acyl, (C₁-C₆)alkyl-CO₂-(C₁-C₆)alkyl, (C₁-C₆)alkyl-OCO-(C₁-C₆)alkyl, (C₁-C₆)alkyl-CO₂-, NCO, R³OCO-, R³NHCO- wherein R³ is hydrogen or (C₁-C₆)alkyl; ((C₁-C₆)alkyl)amino-CO- or phenyl optionally substituted by halo, (C₁-C₆)alkyl, cyano or trifluoromethyl;
Y is nitrogen or CH;
Ar is a group of the formula 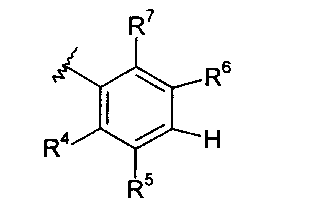 wherein R⁴ and R⁷ are each independently selected from hydrogen, halo, trifluoromethyl, (C₁-C₆)alkoxy optionally substituted with one to three halogen atoms; (C₁-C₆)alkylthio, hydroxy, (C₁-C₆)acyl, cyano, (C₁-C₆)alkyl optionally substituted by amino, (C₁-C₆)alkylamino, ((C₁-C₆)alkyl)₂amino, (C₃-C₇)cycloalkylamino, hydroxy, (C₁-C₆)alkoxy, HCO- H₂NCO-, (C₁-C₆)alkylamino-CO-, ((C₁-C₆)alkyl)₂amino-CO-, (C₃-C₇)cycloalkylamino-CO-, HCO-NH-, (C₁-C₆)alkyl-CO-NH-, HCO-N((C₁-C₆)alkyl)-, (C₁-C₆)alkyl-CO-N((C₁-C₆)alkyl)-, one to three halogen atoms, R⁸O- or R⁸O-CO- wherein R⁸ is hydrogen, (C₁-C₆)alkyl, (C₁-C₆)acyl, (C₁-C₆)alkoxy-CO-, (C₁-C₆)alkylamino-CO-, or ((C₁-C₆)alkyl)₂amino-CO-;
R⁵ and R⁶ are each independently selected from hydrogen, halo, CF₃, (C₁-C₆)alkyl optionally substituted with one to three halogen atoms, (C₁-C₆)alkoxy optionally substituted with one to three halogen atoms, (C₁-C₆)alkylthiol, amino-(CH₂)ₚ-, (C₁-C₆)alkyl-NH-(CH₂)ₚ-, di(C₁-C₆)alkyl-N-(CH₂)ₚ-, (C₃-C₇)cycloalkyl-NH-(CH₂)ₚ-, amino-(C₁-C₆)alkyl-NH-(CH₂)ₚ-, (C₁-C₆)alkyl-NH-(C₁-C₆)alkyl-NH-(CH₂)ₚ-, di(C₁-C₆)alkyl-N((C₁-C₆)aklyl)-(C₁-C₆)alkyl-NH-(CH₂)ₚ-, di(C₁-C₆)alkyl-N-(C₁-C₆)alkyl-N-(CH₂)ₚ-, H₂N-(C=O)-(CH₂)ₚ-, (C₁-C₆)alkyl-HN-(C=O)-(CH₂)ₚ-, di(C₁-C₆)alkyl-N-(C=O)-(CH₂)ₚ, (C₃-C₇)cycloalkyl-NH-(C=O)-(CH₂)ₚ-, R¹³O-(CH₂)ₚ-, R¹³O-(C=O)-(CH₂)ₚ-, H(O=C)-O-, H(O=C)-O-(C₁-C₆)alkyl-, H(O=C)-NH-(CH₂)ₚ-, (C₁-C₆)alkyl-(O=C)-NH-(CH₂)ₚ-, -CHO, H-(C=O)-(CH₂)ₚ-, (C₁-C₆)alkyl-(C=O)-(CH₂)ₚ-, (C₁-C₆)alkyl-(O=C)-N((C₁-C₆)alkyl)-(CH₂)ₚ-, H(O=C)-N((C₁-C₆)alkyl)-(CH₂)ₚ-, HO-(C₁-C₆)alkyl-N((C₁-C₆)alkyl)-(CH₂)ₚ-, (C₁-C₆)alkyl-(C=O)-O-NH-(CH₂)ₚ-, amino-(C₁-C₆)alkyl-(C=O)-O-(CH₂)ₚ-, (C₁-C₆)alkyl-NH-(C₁-C₆)alkyl-(C=O)-O-(CH₂)ₚ-, di(C₁-C₆)alkyl-N-(C₁-C₆)alkyl-(C=O)-O-(CH₂)ₚ-, amino-(C₁-C₆)alkyl-O-(C=O)-(CH₂)ₚ-, (C₁-C₆)alkyl-NH-(C₁-C₆)alkyl-O-(C=O)-(CH₂)ₚ-, di(C₁-C₆)alkyl-N-(C₁-C₆)alkyl-O-(C=O)-(CH₂)ₚ-, hydroxy, hydroxy-(C₁-C₆)alkyl-, hydroxy-(C₁-C₆)alkyl-NH-(CH₂)ₚ-, (C₁-C₆)alkyl-O-(C₁-C₆)alkyl-, -CN, piperidine-(CH₂)ₚ-, pyrrolidine-(CH₂)ₚ-, and 3-pyrroline-(CH₂)ₚ-, wherein said piperidine, pyrrolidine and 3-pyrroline of said piperidine-(CH₂)ₚ-, pyrrolidine-(CH₂)ₚ- and 3-pyrroline-(CH₂)ₚ- moieties may optionally be substituted on any of the ring carbon atoms capable of supporting and additional bond, with a substituent independently selected from halo, CF₃, (C₁-C₆)alkyl optionally substituted with one to three halogen atoms, (C₁-C₆)alkoxy optionally substituted with one to three halogen atoms, (C₁-C₆)alkylthiol, amino-(CH₂)ₚ-, (C₁-C₆)alkyl-NH-(CH₂)ₚ-, di(C₁-C₆)alkyl-N-(CH₂)ₚ-, (C₃-C₇)cycloalkyl-NH-(CH₂)ₚ-, amino-(C₁-C₆)alkyl-NH-(CH₂)ₚ-, (C₁-C₆)alkyl-NH-(C₁-C₆)alkyl-NH-(CH₂)ₚ-, di(C₁-C₆)alkyl-N-(C₁-C₆)alkyl-NH-(CH₂)ₚ-, (C₁-C₆)alkyl-O-(C₁-C₆)alkyl-, di(C₁-C₆)alkyl-N-(C₁-C₆)alkyl-N((C₁-C₆)alkyl)-(CH₂)ₚ-, H₂N-(C=O)-(CH₂)ₚ-, (C₁-C₆)alkyl-HN-(C=O)-(CH₂)ₚ-, di(C₁-C₆)alkyl-N-(C=O)-(CH₂)ₚ, (C₃-C₇)cycloalkyl-NH-(C=O)-(CH₂)ₚ-, R¹³O-(CH₂)ₚ-, R¹³O-(C=O)-(CH₂)ₚ-, H(O=C)-O-, H(O=C)-O-(C₁-C₆)alkyl-, H(O=C)-NH-(CH₂)ₚ-, (C₁-C₆)alkyl-(O=C)-NH-(CH₂)ₚ-, -CHO, H-(C=O)-(CH₂)ₚ-, (C₁-C₆)alkyl-(C=O)-, (C₁-C₆)alkyl-(O=C)-N((C₁-C₆)alkyl)-(CH₂)ₚ-, H(O=C)-N((C₁-C₆)alkyl)-(CH₂)ₚ-, HO-(C₁-C₆)alkyl-N((C₁-C₆)alkyl)-(CH₂)ₚ-, (C₁-C₆)alkyl- (C=O)-O-NH-(CH₂)ₚ-, amino-(C₁-C₆)alkyl-(C=O)-O-(CH₂)ₚ-, (C₁-C₆)alkyl-NH-(C₁-C₆)alkyl-(C=O)-O-(CH₂)ₚ-, di(C₁-C₆)alkyl-N-(C₁-C₆)alkyl-(C=O)-O-(CH₂)ₚ-, hydroxy, hydroxy-(C₁-C₆)alkyl-, hydroxy-(C₁-C₆)alkyl-NH-(CH₂)ₚ-, and -CN;
p is 0 to 6;
or Ar is a group of the formula 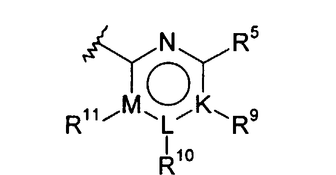 wherein K, L and M are each independently selected from carbon or nitrogen;
R⁵ is defined as above;
R⁹ and R¹⁰ are each independently selected from hydrogen, cyano, (C₁-C₆)alkyl, halo, trifluoromethyl, HCO- or (C₁-C₆)alkoxy;
R¹¹ hydrogen, cyano, halo, trifluoromethyl, (C₁-C₆)alkoxy, HCO-, (C₁-C₆)alkyl optionally substituted by amino, (C₁-C₆)alkylamino or ((C₁-C₆)alkyl)₂amino;
or Ar is a group of the formula 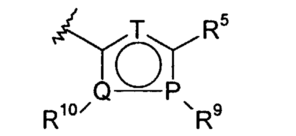 wherein T is CH, N, NH, O or S;
P and Q are each independently selected from carbon, nitrogen, oxygen or sulfur;
R⁵, R⁹ and R¹⁰ are defined as above;
with the proviso that only one of K, L or M can be nitrogen and when K, L or M is nitrogen then its respective R⁹, R¹⁰ or R¹¹ is absent; and
with the proviso that only one of P, Q or T can be oxygen or sulfur and at least one of P, Q or T must be a heteroatom;
which process comprises the steps of:
(a) deprotonating the methyl group at the 2-position of a quinazolin-4-one compound having the formula 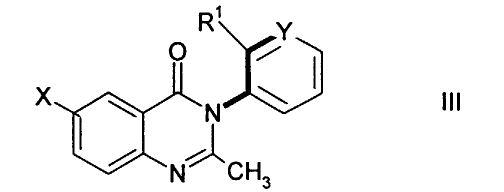 wherein X, Y and R¹ are as defined above, with a base to form the corresponding anion;
b) reacting the anionic intermediate so formed of Step (a) with an aromatic aldehyde, Ar-CHO, to form a quinazolin-4-one derivative of the formula 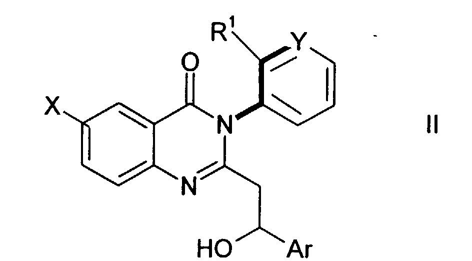 wherein R¹, X, Y and Ar are defined as above; followed by either:
c) dehydrating the product so formed of Step (b) with a dehydrating agent to form a quinazolin-4-one derivative of the formula 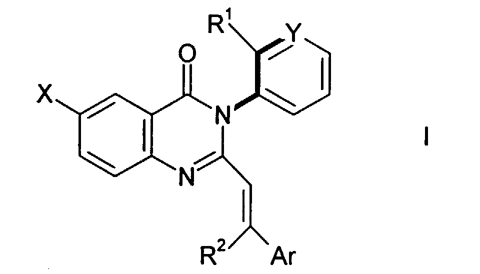 wherein R¹, X, Y and Ar are defined as above and R² is hydrogen; or
d) oxidizing the product so formed of Step (b) with an oxidizing agent to form a quinazolin-4-one derivative of the formula 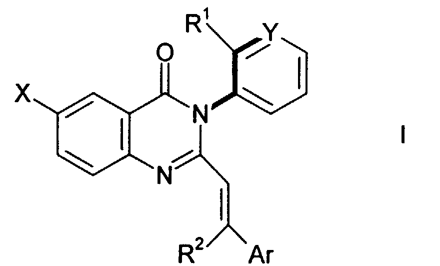 wherein R¹, X, Y and Ar are as defined as above and R₂ is hydroxy.

The present invention also relates to a process for preparing a compound of the formula 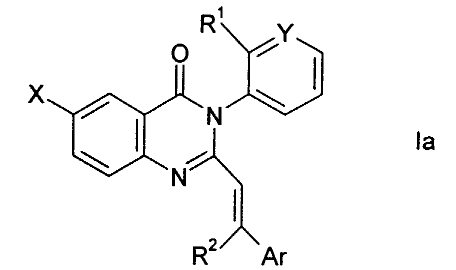 and the pharmaceutically acceptable salts thereof, wherein
R¹ is halo, cyano, (C₁-C₆)alkyl, perfluoro(C₁-C₆)alkyl or -COO(C₁-C₆)alkyl;
R² is hydrogen or hydroxy;
X is hydrogen, hydroxy, halo, trifluoromethyl, nitro, (C₁-C₆)alkyl optionally substituted by (C₁-C₆)alkoxy, hydroxy, amino (C₁-C₆)alkylamino or ((C₁-C₆)alkyl)₂amino; (C₃-C₇)cycloalkyl, (C₁-C₆)alkoxy, (C₁-C₆)acyl, (C₁-C₆)alkyl-CO₂-(C₁-C₆)alkyl, (C₁-C₆)alkyl-OCO-(C₁-C₆)alkyl, (C₁-C₆)alkyl-CO₂-, NCO, R³OCO-, R³NHCO- wherein R³ is hydrogen or (C₁-C₆)alkyl; ((C₁-C₆)alkyl)amino-CO- or phenyl optionally substituted by halo, (C₁-C₆)alkyl, cyano or trifluoromethyl;
Y is nitrogen or CH;
Ar is a group of the formula 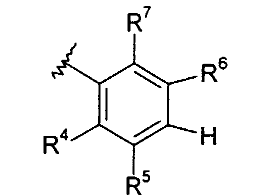 wherein R⁴ and R⁷ are each independently selected from hydrogen, halo, trifluoromethyl, (C₁-C₆)alkoxy optionally substituted with one to three halogen atoms; (C₁-C₆)alkylthio, hydroxy, (C₁-C₆)acyl, cyano, (C₁-C₆)alkyl optionally substituted by amino, (C₁-C₆)alkylamino, ((C₁-C₆)alkyl)₂amino, (C₃-C₇)cycloalkylamino, hydroxy, (C₁-C₆)alkoxy, HCO- H₂NCO-, (C₁-C₆)alkylamino-CO-, ((C₁-C₆)alkyl)₂amino-CO-, (C₃-C₇)cycloalkylamino-CO-, HCO-NH-, (C₁-C₆)alkyl-CO-NH-, HCO-N((C₁-C₆)alkyl)-, (C₁-C₆)alkyl-CO-N((C₁-C₆)alkyl)-, one to three halogen atoms, R⁸O- or R⁸O-CO- wherein R⁸ is hydrogen, (C₁-C₆)alkyl, (C₁-C₆)acyl, (C₁-C₆)alkoxy-CO-, (C₁-C₆)alkylamino-CO-, or ((C₁-C₆)alkyl)₂amino-CO-;
R⁵ and R⁶ are each independently selected from hydrogen, halo, CF₃, (C₁-C₆)alkyl optionally substituted with one to three halogen atoms, (C₁-C₆)alkoxy optionally substituted with one to three halogen atoms, (C₁-C₆)alkylthiol, amino-(CH₂)ₚ-, (C₁-C₆)alkyl-NH-(CH₂)ₚ-, di(C₁-C₆)alkyl-N-(CH₂)ₚ-, (C₃-C₇)cycloalkyl-NH-(CH₂)ₚ-, amino-(C₁-C₆)alkyl-NH-(CH₂)ₚ-, (C₁-C₆)alkyl-NH-(C₁-C₆)alkyl-NH-(CH₂)ₚ-, di(C₁-C₆)alkyl-N((C₁-C₆)alkyl)-(C₁-C₆)alkyl-NH-(CH₂)ₚ-, di(C₁-C₆)alkyl-N-(C₁-C₆)alkyl-N-(CH₂)ₚ-, H₂N-(C=O)-(CH₂)ₚ-, (C₁-C₆)alkyl-HN-(C=O)-(CH₂)ₚ-, di(C₁-C₆)alkyl-N-(C=O)-(CH₂)ₚ, (C₃-C₇)cycloalkyl-NH-(C=O)-(CH₂)ₚ-, R¹³O-(CH₂)ₚ-, R¹³O-(C=O)-(CH₂)ₚ-, H(O=C)-O-, H(O=C)-O-(C₁-C₆)alkyl-, H(O=C)-NH-(CH₂)ₚ-, (C₁-C₆)alkyl-(O=C)-NH-(CH₂)ₚ-, -CHO, H-(C=O)-(CH₂)ₚ-, (C₁-C₆)alkyl-(C=O)-(CH₂)ₚ-, (C₁-C₆)alkyl-(O=C)-N((C₁-C₆)alkyl)-(CH₂)ₚ-, H(O=C)-N((C₁-C₆)alkyl)-(CH₂)ₚ-, HO-(C₁-C₆)alkyl-N((C₁-C₆)alkyl)-(CH₂)ₚ-, (C₁-C₆)alkyl-(C=O)-O-NH-(CH₂)ₚ-, amino-(C₁-C₆)alkyl-(C=O)-O-(CH₂)ₚ-, (C₁-C₆)alkyl-NH-(C₁-C₆)alkyl-(C=O)-O-(CH₂)ₚ-, di(C₁-C₆)alkyl-N-(C₁-C₆)alkyl-(C=O)-O-(CH₂)ₚ-, amino-(C₁-C₆)alkyl-O-(C=O)-(CH₂)ₚ-, (C₁-C₆)alkyl-NH-(C₁-C₆)alkyl-O-(C=O)-(CH₂)ₚ-, di(C₁-C₆)alkyl-N-(C₁-C₆)alkyl-O-(C=O)-(CH₂)ₚ-, hydroxy, hydroxy-(C₁-C₆)alkyl-, hydroxy-(C₁-C₆)alkyl-NH-(CH₂)ₚ-, (C₁-C₆)alkyl-O-(C₁-C₆)alkyl-, -CN, piperidine-(CH₂)ₚ-, pyrrolidine-(CH₂)ₚ-, and 3-pyrroline-(CH₂)ₚ-, wherein said piperidine, pyrrolidine and 3-pyrroline of said piperidine-(CH₂)ₚ-, pyrrolidine-(CH₂)ₚ- and 3-pyrroline-(CH₂)ₚ- moieties may optionally be substituted on any of the ring carbon atoms capable of supporting and additional bond, with a substituent independently selected from halo, CF₃, (C₁-C₆)alkyl optionally substituted with one to three halogen atoms, (C₁-C₆)alkoxy optionally substituted with one to three halogen atoms, (C₁-C₆)alkylthiol, amino-(CH₂)ₚ-, (C₁-C₆)alkyl-NH-(CH₂)ₚ-, di(C₁-C₆)alkyl-N-(CH₂)ₚ-, (C₃-C₇)cycloalkyl-NH(-CH₂)ₚ-, amino-(C₁-C₆)alkyl-NH-(CH₂)ₚ-, (C₁-C₆)alkyl-NH-(C₁-C₆)alkyl-NH-(CH₂)ₚ-, -di(C₁-C₆)alkyl-N-C₁-C₆)alkyl-NH-(CH₂)ₚ-, (C₁-C₆)alkyl-O-(C₁-C₆)alkyl-, di(C₁-C₆)alkyl-N-(C₁-C₆)alkyl-N((C₁-C₆)alkyl)-(CH₂)ₚ-, H₂N-(C=O)-(CH₂)ₚ-, (C₁-C₆)alkyl-HN-(C=O)-(CH₂)ₚ-, di(C₁-C₆)alkyl-N-(C=O)-(CH₂)ₚ, (C₃-C₇)cycloalkyl-NH-(C=O)-(CH₂)ₚ-, R¹³O-(CH₂)ₚ-, R¹³O-(C=O)-(CH₂)ₚ-, H(O=C)-O-, H(O=C)-O-(C₁-C₆)alkyl-, H(O=C)-NH-(CH₂)ₚ-, (C₁-C₆)alkyl-(O=C)-NH-(CH₂)ₚ-, -CHO, H-(C=O)-(CH₂)ₚ-, (C₁-C₆)alkyl-(C=O)-, (C₁-C₆)alkyl-(O=C)-N((C₁-C₆)alkyl)-(CH₂)ₚ-, H(O=C)-N((C₁-C₆)alkyl)-(CH₂)ₚ-, HO-(C₁-C₆)alkyl-N((C₁-C₆)alkyl)-(CH₂)ₚ-, (C₁-C₆)alkyl- (C=O)-O-NH-(CH₂)ₚ-, amino-(C₁-C₆)alkyl-(C=O)-O-(CH₂)ₚ-, (C₁-C₆)alkyl-NH-(C₁-C₆)alkyl-(C=O)-O-(CH₂)ₚ-, di(C₁-C₆)alkyl-N-(C₁-C₆)alkyl-(C=O)-O-(CH₂)ₚ-, hydroxy, hydroxy-(C₁-C₆)alkyl-, hydroxy-(C₁-C₆)alkyl-NH-(CH₂)ₚ-, and -CN;
p is 0 to 6;
or Ar is a group of the formula 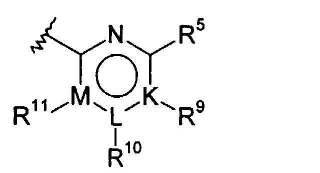 wherein K, L and M are each independently selected from carbon or nitrogen;
R⁵ is defined as above;
R⁹ and R¹⁰ are each independently selected from hydrogen, cyano, (C₁-C₆)alkyl, halo, trifluoromethyl, HCO- or (C₁-C₆)alkoxy;
R¹¹ hydrogen, cyano, halo, trifluoromethyl, (C₁-C₆)alkoxy, HCO-, (C₁-C₆)alkyl optionally substituted by amino, (C₁-C₆)alkylamino or ((C₁-C₆)alkyl)₂amino;
or Ar is a group of the formula 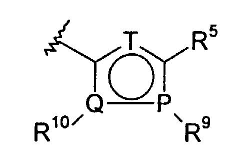 wherein T is CH, N, NH, O or S;
P and Q are each independently selected from carbon, nitrogen, oxygen or sulfur;
R⁵, R⁹ and R¹⁰ are defined as above;
with the proviso that only one of K, L or M can be nitrogen and when K, L or M is nitrogen then its respective R⁹, R¹⁰ or R¹¹ is absent; and
with the proviso that only one of P, Q or T can be oxygen or sulfur and at least one of P, Q or T must be a heteroatom;
comprising either (a) dehydrating the quinazolin-4-one derivative of the formula 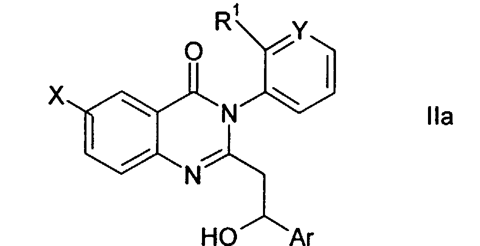 with a dehydration agent to form the compound of formula **Ia**, wherein R¹, X, Y and Ar are defined as above and R² is hydrogen; or
(b) oxidizing the compound of formula **IIa** with an oxidizing agnet to form the compound of formula **Ia**, wherein R¹, X, Y and Ar are defined as above and R² is hydroxy.

A process for preparing the compound of formula **Ia**, wherein the dehydration agent is selected from acetic anhydride, trifluoroacetic anhydride, acetyl chloride, methanesulfonyl chloride, Burgess salt, thienyl chloride, phosphorus triichloride or phosphorus oxychloride.

A process for preparing the compound of formula **Ia**, wherein the oxidizing agent is selected from chromium trioxide, pyridinium dichromate, pyridinium chlorochromate, dicyclohexylcarbodiimide or ethyl 2-dimethylaminoethyl carbodiimide.

The present invention also relates to a process according to claim 1, wherein the compound of the formula 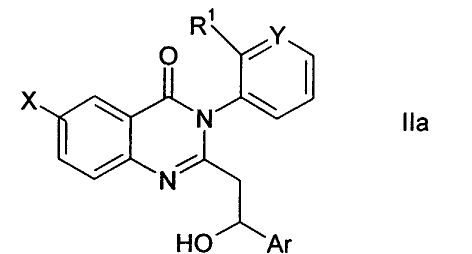 is formed by (a) deprotonating the methyl group at the 2-position of a quinazolin-4-one compound having the formula 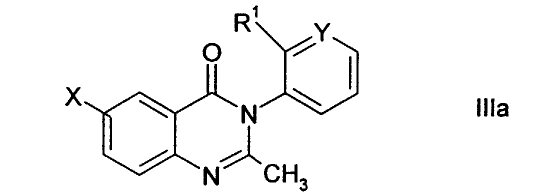 wherein X, Y and R¹ are as defined above, with a base to form the corresponding anion; and (b) reacting the anionic intermediate so formed with an aromatic aldehyde of the formula Ar-CHO, wherein Ar is defined as above, to form the compound of the formula **IIa**.

A process for preparing the compound of formula **IIa**, wherein the base is selected from lithium diisopropylamide, lithium hexamethyldisilylamide, sodium hexamethyldisilylamide, potassium hexamethyldisilylamide or lithium 2,2,6,6-tetramethylpiperidide.

The present invention also relates to a process for preparing a compound of the formula 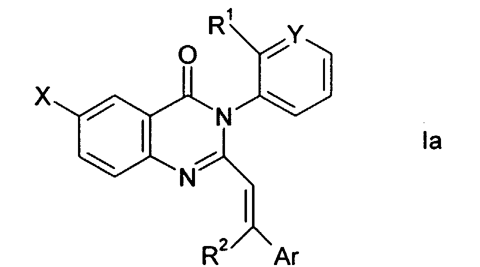 and the pharmaceutically acceptable salts thereof, wherein
R¹ is halo, cyano, (C₁-C₆)alkyl, perfluoro(C₁-C₆)alkyl or --COO(C₁-C₆)alkyl;
R² is hydrogen or hydroxy;
X is hydrogen, hydroxy, halo, trifluoromethyl, nitro, (C₁-C₆)alkyl optionally substituted by (C₁-C₆)alkoxy, hydroxy, amino (C₁-C₆)alkylamino or ((C₁-C₆)alkyl)₂amino; (C₃-C₇)cycloalkyl, (C₁-C₆)alkoxy, (C₁-C₆)acyl, (C₁-C₆)alkyl-CO₂-(C₁-C₆)alkyl, (C₁-C₆)alkyl-OCO-(C₁-C₆)alkyl, (C₁-C₆)alkyl-CO₂-, NCO, R³OCO-, R³NHCO- wherein R³ is hydrogen or (C₁-C₆)alkyl; ((C₁-C₆)alkyl)amino-CO- or phenyl optionally substituted by halo, (C₁-C₆)alkyl, cyano or trifluoromethyl;
Y is nitrogen or CH;
Ar is a group of the formula 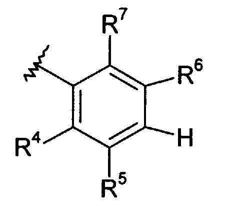 wherein R⁴ and R⁷ are each independently selected from hydrogen, halo, trifluoromethyl, (C₁-C₆)alkoxy optionally substituted with one to three halogen atoms; (C₁-C₆)alkylthio, hydroxy, (C₁-C₆)acyl, cyano, (C₁-C₆)alkyl optionally substituted by amino, (C₁-C₆)alkylamino, ((C₁-C₆)alkyl)₂amino, (C₃-C₇)cycloalkylamino, hydroxy, (C₁-C₆)alkoxy, HCO- H₂NCO-, (C₁-C₆)alkylamino-CO-, ((C₁-C₆)alkyl)₂amino-CO-, (C₃-C₇)cycloalkylamino-CO-, HCO-NH-, (C₁-C₆)alkyl-CO-NH-, HCO-N((C₁-C₆)alkyl)-, (C₁-C₆)alkyl-CO-N((C₁-C₆)alkyl)-, one to three halogen atoms, R⁸O- or R⁸O-CO- wherein R⁸ is hydrogen, (C₁-C₆)alkyl, (C₁-C₆)acyl, (C₁-C₆)alkoxy-CO-, (C₁-C₆)alkylamino-CO-, or ((C₁-C₆)alkyl)₂amino-CO-;
R⁵ and R⁶ are each independently selected from hydrogen, halo, CF₃, (C₁-C₆)alkyl optionally substituted with one to three halogen atoms, (C₁-C₆)alkoxy optionally substituted with one to three halogen atoms, (C₁-C₆)alkythiol, amino-(CH₂)ₚ-, (C₁-C₆)alkyl-NH-(CH₂)ₚ-, di(C₁-C₆)alkyl-N-(CH₂)ₚ-, (C₃-C₇)cycloalkyl-NH-(CH₂)ₚ-, amino-(C₁-C₆)alkyl-NH-(CH₂)ₚ-, (C₁-C₆)alkyl-NH-(C₁-C₆)alkyl-NH-(CH₂)ₚ-, di(C₁-C₆)alkyl-N((C₁-C₆)alkyl)-(C₁-C₆)alkyl-NH-(CH₂)ₚ-, di(C₁-C₆)alkyl-N-(C₁-C₆)alkyl-N-(CH₂)ₚ-, H₂N-(C=O)-(CH₂)ₚ-, (C₁-C₆)alkyl-HN-(C=O)-(CH₂)ₚ-, di(C₁-C₆)alkyl-N-(C=O)-(CH₂)ₚ, (C₃-C₇)cycloalkyl-NH-(C=O)-(CH₂)ₚ-, R¹³O-(CH₂)ₚ-, R¹³O-(C=O)-(CH₂)ₚ-, H(O=C)-O-, H(O=C)-O-(C₁-C₆)alkyl-, H(O=C)-NH-(CH₂)ₚ-, (C₁-C₆)alkyl-(O=C)-NH-(CH₂)ₚ-, -CHO, H-(C=O)-(CH₂)ₚ-, (C₁-C₆)alkyl-(C=O)-(CH₂)ₚ-, (C₁-C₆)alkyl-(O=C)-N((C₁-C₆)alkyl)-(CH₂)ₚ-, H(O=C)-N((C₁-C₆)alkyl)-(CH₂)ₚ-, HO-(C₁-C₆)alkyl-N((C₁-C₆)alkyl)-(CH₂)ₚ-, (C₁-C₆)alkyl-(C=O)-O-NH-(CH₂)ₚ-, amino-(C₁-C₆)alkyl-(C=O)-O-(CH₂)ₚ-, (C₁-C₆)alkyl-NH-(C₁-C₆)alkyl-(C=O)-O-(CH₂)ₚ-, di(C₁-C₆)alkyl-N-(C₁-C₆)alkyl-(C=O)-O-(CH₂)ₚ-, amino-(C₁-C₆)alkyl-O-(C=O)-(CH₂)ₚ-, (C₁-C₆)alkyl-NH-(C₁-C₆)alkyl-O-(C=O)-(CH₂)ₚ-, di(C₁-C₆)alkyl-N-(C₁-C₆)alkyl-O-(C=O)-(CH₂)ₚ-, hydroxy, hydroxy-(C₁-C₆)alkyl-, hydroxy-(C₁-C₆)alkyl-NH-(CH₂)ₚ-, (C₁-C₆)alkyl-O-(C₁-C₆)alkyl-, -CN, piperidine-(CH₂)ₚ-, pyrrolidine-(CH₂)ₚ-, and 3-pyrroline-(CH₂)ₚ-, wherein said piperidine, pyrrolidine and 3-pyrroline of said piperidine-(CH₂)ₚ-, pyrrolidine-(CH₂)ₚ- and 3-pyrroline-(CH₂)ₚ- moieties may optionally be substituted on any of the ring carbon atoms capable of supporting and additional bond, with a substituent independently selected from halo, CF₃, (C₁-C₆)alkyl optionally substituted with one to three halogen atoms, (C₁-C₆)alkoxy optionally substituted with one to three halogen atoms, (C₁-C₆)alkylthiol, amino-(CH₂)ₚ-, (C₁-C₆)alkyl-NH-(CH₂)ₚ-, di(C₁-C₆)alkyl-N-(CH₂)ₚ-, (C₃-C₇)cycloalkyl-NH-(CH₂)ₚ-, amino-(C₁-C₆)alkyl-NH-(CH₂)ₚ-, (C₁-C₆)alkyl-NH-(C₁-C₆)alkyl-NH-(CH₂)ₚ-, di(C₁-C₆)alkyl-N-(C₁-C₆)alkyl-NH-(CH₂)ₚ-, (C₁-C₆)alkyl-O-(C₁-C₆)alkyl-, di(C₁-C₆)alkyl-N-(C₁-C₆)alkyl-N((C₁-C₆)alkyl)-(CH₂)ₚ-, H₂N-(C=O)-(CH₂)ₚ-, (C₁-C₆)alkyl-HN-(C=O)-(CH₂)ₚ-, di(C₁-C₆)alkyl-N-(C=O)-(CH₂)ₚ, (C₃-C₇)cycloalkyl-NH-(C=O)-(CH₂)ₚ-, R¹³O-(CH₂)ₚ-, R¹³O-(C=O)-(CH₂)ₚ-, H(O=C)-O-, H(O=C)-O-(C₁-C₆)alkyl-, H(O=C)-NH-(CH₂)ₚ-, (C₁-C₆)alkyl-(O=C)-NH-(CH₂)ₚ-, -CHO, H-(C=O)-(CH₂)ₚ-, (C₁-C₆)alkyl-(C=O)-, (C₁-C₆)alkyl-(O=C)-N((C₁-C₆)alkyl)-(CH₂)ₚ-, H(O=C)-N((C₁-C₆)alkyl)-(CH₂)ₚ-, HO-(C₁-C₆)alkyl-N((C₁-C₆)alkyl)-(CH₂)ₚ-, (C₁-C₆)alkyl- (C=O)-O-NH-(CH₂)ₚ-, amino-(C₁-C₆)alkyl-(C=O)-O-(CH₂)ₚ-, (C₁-C₆)alkyl-NH-(C₁-C₆)alkyl-(C=O)-O-(CH₂)ₚ-, di(C₁-C₆)alkyl-N-(C₁-C₆)alkyl-(C=O)-O-(CH₂)ₚ-, hydroxy, hydroxy-(C₁-C₆)alkyl-, hydroxy-(C₁-C₆)alkyl-NH-(CH₂)ₚ-, and -CN;
p is 0 to 6;
or Ar is a group of the formula 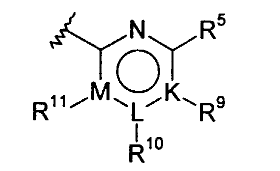 wherein K, L and M are each independently selected from carbon or nitrogen;
R⁵ is defined as above;
R⁹ and R¹⁰ are each independently selected from hydrogen, cyano, (C₁-C₆)alkyl, halo, trifluoromethyl, HCO- or (C₁-C₆)alkoxy;
R¹¹ hydrogen, cyano, halo, trifluoromethyl, (C₁-C₆)alkoxy, HCO-, (C₁-C₆)alkyl optionally substituted by amino, (C₁-C₆)alkylamino or ((C₁-C₆)alkyl)₂amino;
or Ar is a group of the formula 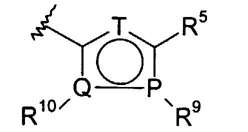 wherein T is CH, N, NH, O or S;
P and Q are each independently selected from carbon, nitrogen, oxygen or sulfur;
R⁵, R⁹ and R¹⁰ are defined as above;
with the proviso that only one of K, L or M can be nitrogen and when K, L or M is nitrogen then its respective R⁹, R¹⁰ or R¹¹ is absent; and
with the proviso that only one of P, Q or T can be oxygen or sulfur and at least one of P, Q or T must be a heteroatom;
which process comprises the steps of:
(a) deprotonating the methyl group at the 2-position of a quinazolin-4-one compound having the formula 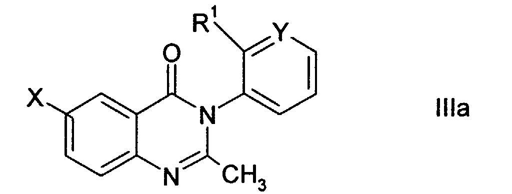 wherein X, Y and R¹ are as defined above, with a base to form the corresponding anion;
b) reacting the anionic intermediate so formed of Step (a) with an aromatic aldehyde, Ar-CHO, to form a quinazolin-4-one derivative of the formula 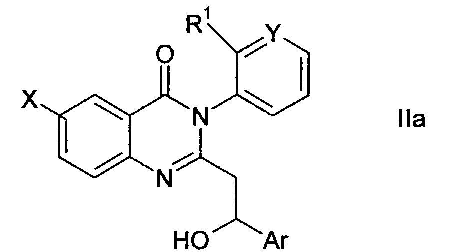 wherein R¹, X, Y and Ar are defined as above; followed by either:
c) dehydrating the product so formed of Step (b) with a dehydration agent to form a quinazolin-4-one derivative of the formula 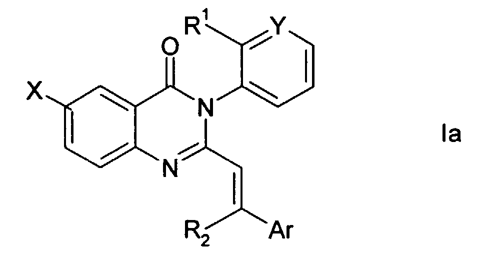 wherein R¹, X, Y and Ar are defined as above and R² is hydrogen; or
d) oxidizing the product so formed of Step (b) with an oxidizing agent to form a quinazolin-4-one derivative of the formula 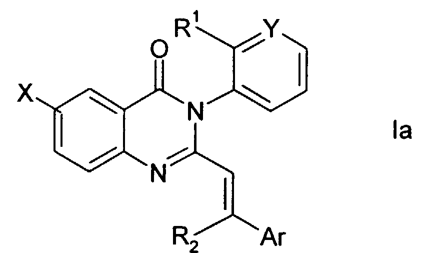 wherein R¹, X, Y and Ar are as defined as above and R² is hydroxy.

The present invention also relates to a compound of the formula 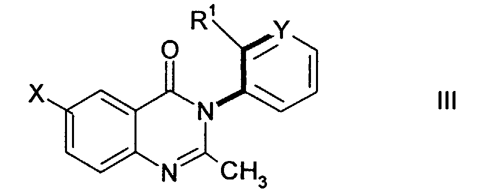 wherein R¹ is halo, cyano, (C₁-C₆)alkyl, perfluoro(C₁-C₆)alkyl or -COO(C₁-C₆)alkyl;
X is hydrogen, hydroxy, halo, trifluoromethyl, nitro, (C₁-C₆)alkyl optionally substituted by (C₁-C₆)alkoxy, hydroxy, amino (C₁-C₆)alkylamino or ((C₁-C₆)alkyl)₂amino; (C₃-C₇)cycloalkyl, (C₁-C₆)alkoxy, (C₁-C₆)acyl, (C₁-C₆)alkyl-CO₂-(C₁-C₆)alkyl, (C₁-C₆)alkyl-OCO-(C₁-C₆)alkyl, (C₁-C₆)alkyl-CO₂-, NCO, R³OCO-, R³NHCO- wherein R³ is hydrogen or (C₁-C₆)alkyl; ((C₁-C₆)alkyl)amino-CO- or phenyl optionally substituted by halo, (C₁-C₆)alkyl, cyano or trifluoromethyl;
Y is nitrogen or CH.

The present invention also relates to a compound of the formula 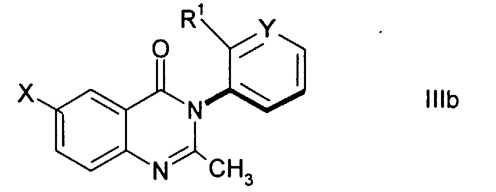 wherein R¹ is halo, cyano, (C₁-C₆)alkyl, perfuoro(C₁-C₆)alkyl or -COO(C₁-C₆)alkyl;
X is hydrogen, hydroxy, halo, trifluoromethyl, nitro, (C₁-C₆)alkyl optionally substituted by (C₁-C₆)alkoxy, hydroxy, amino (C₁-C₆)alkylamino or ((C₁-C₆)alkyl)₂amino; (C₃-C₇)cycloalkyl, (C₁-C₆)alkoxy, (C₁-C₆)acyl, (C₁-C₆)alkyl-CO₂-(C₁-C₆)alkyl, (C₁-C₆)alkyl-OCO-(C₁-C₆)alkyl, (C₁-C₆)alkyl-CO₂-, NCO, R³OCO-, R³NHCO- wherein R³ is hydrogen or (C₁-C₆)alkyl; ((C₁-C₆)alkyl)amino-CO- or phenyl optionally substituted by halo, (C₁-C₆)alkyl, cyano or trifluoromethyl;
Y is nitrogen or CH.

### Detailed Description of the Inveniton

The following reaction Scheme illustrates the preparation of the compounds of the present invention. Unless otherwise indicated R¹, R², X, Y and Ar in the reaction Schemes and the discussion that follow are defined as above.

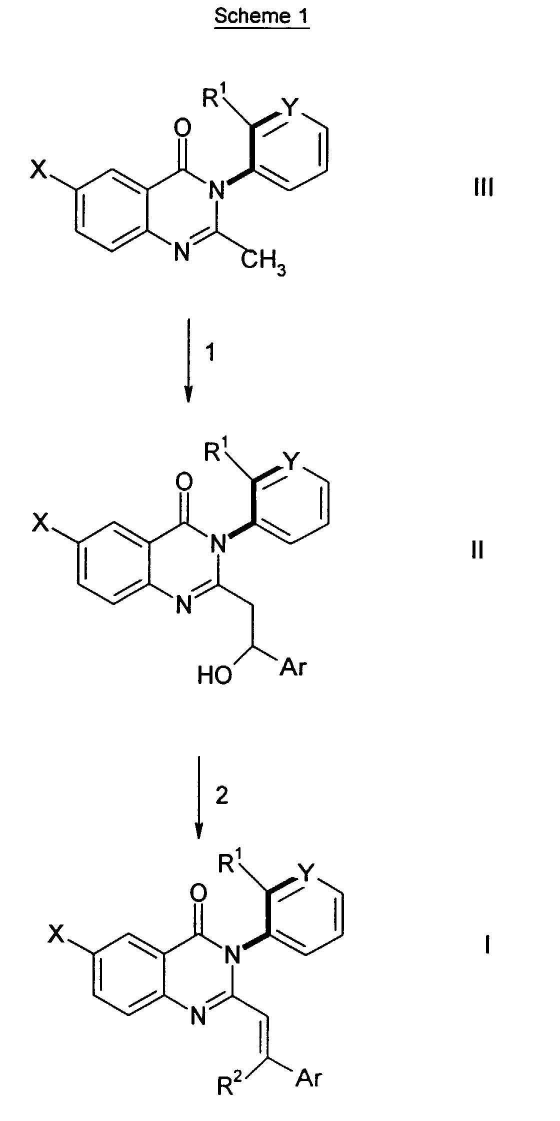

The compounds of formula **III**, the starting materials used in Scheme 1, may be prepared as described in patent applications WO 97/43276 and EP 807633, both of which are incorporated herein by reference in their entirety. The compounds of formula (III) may also be prepared according to the methodology described in Miyashita, et al., Heterocycles, 42 (2), 691-699 (1996), incorporated herein by reference inits entirety.

Single atropisomers may be equilibrated by heating. Therefore, synthetic reaction conditions which are conducted below the equilibrating temperatures of the atropisomers may allow atropisomerically pure compounds of formula **III** to be converted to compounds of formula I with retention of atropisomeric purity. A significant advantage of the present invention relates to the low temperatures required to prepare compounds of formula **I**. Thus, single atropisomers of formula **I** are obtained from single atropisomers of formula **III** by employing the following process.

Racemic compounds of formula **III** may be purified into their component atropisomers by any of several methods. For example, separation may be achieved by high pressure liquid chromatography (HPLC) using a chiral liquid chromatography column. When compounds **III** contain a basic or acidic functional group, the atropisomers may be separated by recrystallization of their diasteromeric salts with chiral acids or bases, respectively. Occasionally hot crystallization (equilibrating conditions) in the presence of a chiral acid (or base) results in selective crystallization of a single atropisomer salt.

In reaction 1 of Scheme 1, the 2-methyl quinazolin-4-one compound of formula **III** is converted to the corresponding compound of formula **II** by deprotonating **III**, at the 2-methyl position, in a polar aprotic solvent, such as tetrahydrofuran, ether, dioxane or dimethoxyethane, with a base, such as lithium diisopropylamide, lithium hexamethyldisilylamide, sodium hexamethyldisilylamide, potassium hexamethyldisilylamide or lithium 2,2,6,6-tetramethylpiperidide, which is of sufficient strength to effect deprotonation and is contemporaneously compatible with other functional groups which may be present in the molecule as well as the reaction solvent in which the deprotonation step is effected. The reaction mixture is stirred at a temperature between about -100°C to about -25°C, preferably about -78°C. The anionic intermediate so formed is then reacted with an aromatic aldehyde of the formula, Ar-CHO, wherein Ar is defined as above. The aromatic aldehyde can be added to the anion solution (normal addition) or the anion solution can be added to the aromatic aldehyde (inverse addition). While both methods can be used to produce compounds of formula **II**, inverse addition is preferred. The resulting mixture is stirred at a temperature between about 0°C to about -100°C, preferably about -78°C, for a time period between about 5 minutes to about 5 hours, preferably about 30 minutes.

In reaction 2 of Scheme 1, the compound of formula **II** is converted to the corresponding 2-[2-(aryl or heteroaryl)-2-hydroxy-vinyl]quinazolin-4-one compound of formula **I**, wherein R² is hydroxy, by oxidizing the compound of formula **II**, with a suitable oxidizing reagent, such as chromium trioxide, pyridinium dichromate, pyridinium chlorochromate, dimethyl sulfoxide and any of the various diimides (for example dicyclohexylcarbodiimide or ethyl 2-dimethylaminoethyl carbodiimide). Suitable solvents for these reactions will be somewhat dependent on the particular oxidant and include methylenechloride, pyridine, DMSO, benzene, toluene. Additives such as trifluoroacetic acid, pyridine, and/or phenyl phosphoric acid may be added to facilitate the oxidation. Reactions may be carried out from -20°C to about 100°C, preferably at ambient temperature to about 50°C.

The compound of formula **II** is converted to the corresponding 2-[2-(aryl or heteroaryl)-vinyl]quinazolin-4-one compound of formula **I**, wherein R² is hydrogen, by dehydrating **II**. Dehydration may be carried out in dioxane, tetrahydrofuran ether, methylene chloride, dichloroethane, preferably dioxane with a suitable dehydration agent such as acetic anhydride, trifluoroacetic anhydride, acetyl chloride, methanesulfonyl chloride, Burgess salt (Burgess et al., J. Org. Chem. 1973, 38, 26), thienyl chloride, phosphorus trichloride, phosphorus oxychloride, preferably trifluoroacetic acid. The reaction is generally carried out at a temperature of about-20°C to about 100°C, preferably ambient temperature to about 50°C.

Racemic compounds of formula **I** may also be obtained from racemic compounds of formula **III** by employing the process decribed above in Scheme 1. In this case, the temperature ranges, in reaction 2, of Scheme 1, may be increased above the equilibrating temperatures of the atropisomers since isomerization would not be an issue.

The compounds of the present invention that are basic in nature are capable of forming a wide variety of different salts with various inorganic and organic acids. Although such salts must be pharmaceutically acceptable for administration to animals, it is often desirable in practice to initially isolate the compound of the present invention from the reaction mixture as a pharmaceutically unacceptable salt and then simply convert the latter back to the free base compound by treatment with an alkaline reagent and subsequently convert the latter free base to a pharmaceutically acceptable acid addition salt The acid addition salts of the base compounds of this invention are readily prepared by treating the base compound with a substantially equivalent amount of the chosen mineral or organic acid in an aqueous solvent medium or in a suitable organic solvent, such as methanol or ethanol. Upon careful evaporation of the solvent, the desired solid salt is readily obtained. The desired acid salt can also be precipitated from a solution of the free base in an organic solvent by adding to the solution an appropriate mineral or organic acid.

Those compounds of the present invention that are acidic in nature, are capable of forming base salts with various pharmacologically acceptable cations. Examples of such salts include the alkali metal or alkaline-earth metal salts and particularly, the sodium and potassium salts. These salts are all prepared by conventional techniques. The chemical bases which are used as reagents to prepare the pharmaceutically acceptable base salts of this invention are those which form non-toxic base salts with the acidic compounds of the present invention. Such non-toxic base salts include those derived from such pharmacologically acceptable cations as sodium, potassium calcium and magnesium, etc. These salts can easily be prepared by treating the corresponding acidic compounds with an aqueous solution containing the desired pharmacologically acceptable cations, and then evaporating the resulting solution to dryness, preferably under reduced pressure. Alternatively, they may also be prepared by mixing lower alkanolic solutions of the acidic compounds and the desired alkali metal alkoxide together, and then evaporating the resulting solution to dryness in the same manner as before. In either case, stoichiometric quantities of reagents are preferably employed in order to ensure completeness of reaction and maximum yields of the desired final product.

The present invention is illustrated by the following examples, but it is not limited to the details thereof.

### Example 1

### 3-(2-Chloro-phenyl)-6-fluoro-2-[2-(2-fluoro-phenyl)-2-hydroxy-ethyl]-3H-quinazolin-4-one

A solution of diisopropylamine (0.60 mL, 4.57 mmol) in tetrahydrofuran (27 mL) was chilled to -78° C and butyllithium (1.3 mL, 3.25 mmol, 2.5 N in hexanes) was added dropwise. The solution was stirred 20 min and then a solution 3-(2-chloro-phenyl)-6-fluoro-2-methyl-3H-quinazolin-4-one (1.04 g, 3.6 mmol) in tetrahydrofuran (7 mL) was added dropwise. The solution became intense red and was stirred 30 min. In a separate vessel a solution of 2-fluorobenzaldehyde (0.575 mL, 5.46 mmol) in tetrahydrofuran (20 mL) was prepared and chilled to -78° C. The cold red anion solution was added to the cold 2-fluorobenzaldehyde solution via canula over about one minute. The resulting mixture was stirred 30 min at -78° C and then was allowed to warm to ambient temperature. The reaction was quenched with saturated aqueous sodium bicarbonate and repeatedly extracted with ethyl acetate. The combined organic layer was concentrated and the residue was taken up in a mixture of water (50 mL), ethyl acetate (10 mL) and saturated aqueous sodium bisulfite (50 mL). The mixture was stirred 1 h, then the layers were separated and the aqueous layer was repeatedly extracted with ethyl acetate. The combined extracts were washed with water and brine, dried over magnesium sulfate and concentrated. The residue was flash chromatographed on silica gel (45 x 150 mm) with elution proceeding as follows: 20% ethyl acetate / hexane (500 mL), nil; 30% ethyl acetate / hexane (500 mL) and 40% ethyl acetate / hexane (500 mL) 0.514 g (38%) of 3-(2-chloro-phenyl)-6-fluoro-2-[2-(2-fluoro-phenyl)-2-hydroxy-ethyl]-3H-quinazolin-4-one as a viscous oily mixture of diastereomers which had: NMR δ 7.90 (dd, J = 3, 8 Hz, 1 H), 7.75 (dd, J = 5, 9 Hz, 1 H), 7.60-7.48 (m, 3 H), 7.46-7.30 M, 2 H), 7.24-7.17 (m, 1 H), 7.12-7.07 (m, 2 H), 6.97-6.89 (m, 1 H), 5.60-5.54 (m, 2 H), 2.73-2.55 (m, 2 H); APCI MS m/z = 413 (P⁺¹). The product was suitable for use without further purification.

### Example 2

### 3-(2-Chloro-phenyl)-2-[2-(6-diethylaminomethyl-pyridin-2-yl)-2-hydroxy-ethyl]-6-fluoro-3H-quinazolin-4-one

A solution of diisopropylamine (0.29 mL, 2.21 mmol) in tetrahydrofuran (27 mL) was chilled to -78° C and butyllithium (0.625 mL, 1.56 mmol, 2.5 N in hexanes) was added dropwise. The solution was stirred 20 min and then a solution 3-(2-chloro-phenyl)-6-fluoro-2-methyl-3H-quinazolin-4-one (0.485 g, 1.67 mmol) in tetrahydrofuran (7 mL) was added dropwise. The solution became intense red and was stirred 30 min. In a separate vessel a solution of 6-diethylaminomethyl-pyridine-2-carboxaldehyde (0.501 g, 2.6 mmol) in tetrahydrofuran (20 mL) was prepared and chilled to -78° C. The cold red anion solution was added to the cold 6-diethylaminomethyl-pyridine-2-carboxaldehyde solution via canula over about one minute. The resulting mixture was stirred 30 min at -78° C and then was allowed to warm to ambient temperature. The reaction was quenched with saturated aqueous sodium bicarbonate and repeatedly extracted with ethyl acetate. The combined organic layer was concentrated and the residue was taken up in a mixture of water (50 mL), ethyl acetate (10 mL) and saturated aqueous sodium bisulfite (50 mL). The mixture was stirred 1 h, then the layers were separated and the aqueous layer was repeatedly extracted with ethyl acetate. The combined extracts were washed with water and brine, dried over magnesium sulfate and concentrated. The residue was flash chromatographed on silica gel (40 x 125 mm) with elution proceeding as follows: 2% methanol / methylene chloride (500 mL), nil; 3% methanol / methylene chloride (500 mL), nil; 4% methanol / methylene chloride (250 mL), unweighed recovered 3-(2-chloro-phenyl)-6-fluoro-2-methyl-3H-quinazolin-4-one; 5% methanol / methylene chloride (250 mL), nil; 6% methanol / methylene chloride (250 mL) and 10% methanol / methylene chloride (1000 mL) and 10% methanol / 1% ammonium hydroxide / methylene chloride (1000 mL), 0.295 g (39%) of 3-(2-chloro-phenyl)-2-[2-(6-diethylaminomethyl-pyridin-2-yl)-2-hydroxy-ethyl]-6-fluoro-3H-quinazolin-4-one as a viscous oily mixture of diastereomers which had: APCI MS m/z = 481 (P⁺¹). The product was suitable for use without further purification.

### Example 3

### 3-(2-Chloro-phenyl)-2-[2-(6-pyrrolidin-1-ylmethyl-pyridin-2-yl)-2-hydroxy-ethyl]-6-fluoro-3H-quinazolin-4-one

A solution of diisopropylamine (0.29 mL, 2.21 mmol) in tetrahydrofuran (27 mL) was chilled to -78° C and butyllithium (0.625 mL, 1.56 mmol, 2.5 N in hexanes) was added dropwise. The solution was stirred 20 min and then a solution 3-(2-chloro-phenyl)-6-fluoro-2-methyl-3H-quinazolin-4-one (0.48 g, 1.66 mmol) in tetrahydrofuran (7 mL) was added dropwise. The solution became intense red and was stirred 30 min. In a separate vessel a solution of 6-pyrrolidin-1-ylmethyl-pyridine-2-carboxaldehyde (0.50 g, 2.62 mmol) in tetrahydrofuran (20 mL) was prepared and chilled to -78° C. The cold red anion solution was added to the cold 6-pyrrolidin-1-ylmethyl-pyridine-2-carboxaldehyde solution via canula over about one minute. The resulting mixture was stirred 30 min at -78° C and then was allowed to warm to ambient temperature. The reaction was quenched with saturated aqueous sodium bicarbonate and repeatedly extracted with ethyl acetate. The combined organic layer was concentrated and the residue was taken up in a mixture of water (50 mL), ethyl acetate (10 mL) and saturated aqueous sodium bisulfite (50 mL). The mixture was stirred 1 h, then the layers were separated and the aqueous layer was repeatedly extracted with ethyl acetate. The combined extracts were washed with water and brine, dried over magnesium sulfate and concentrated. The residue was flash chromatographed on silica gel (40 x 100 mm) with elution proceeding as follows: 3% methanol / 0.3% ammonium hydroxide / methylene chloride (250 mL), nil; 4% methanol / 0.4% ammonium hydroxide / methylene chloride (250 mL), recovered unweighed 3-(2-chloro-phenyl)-6-fluoro-2-methyl-3H-quinazolin-4-one; 5% methanol / 0.5% ammonium hydroxide / methylene chloride (250 mL), nil; 6% methanol / 0.6% ammonium hydroxide / methylene chloride (250 mL) and 7% methanol / 0.7% ammonium hydroxide / methylene chloride (250 mL), 0.26 g (35%) of 3-(2-chloro-phenyl)-2-[2-(6-pyrrolidin-1-ylmethyl-pyridin-2-yl)-2-hydroxy-ethyl]-6-fluoro-3H-quinazolin-4-one as a viscous oily mixture of diastereomers which had: APCI MS m/z = 479 (P⁺¹). The product was suitable for use without further purification.

### Example 4

### 3-(2-Chloro-phenyl)-6-fluoro-2-[2-(2-fluoro-phenyl)-2-hydroxy-vinyl]-3H-quinazolin-4-one

To a mixture of trifluoroacetic acid (0.005 mL), pyridine (0.010 mL), benzene (1 mL), and dimethyl sulfoxide (1 mL) was added 3-(2-chloro-phenyl)-6-fluoro-2-[2-(2-fluoro-phenyl)-2-hydroxy-ethyl]-3H-quinazolin-4-one (0.47 g, 0.114 mmol, product of example 1). After 2 min, ethyl 2-dimethylaminoethyl carbodiimide (0.134 g, 0.7 mmol) was added all at once. The mixture was stirred at ambient temperature ovemight. The reaction was diluted with water and repeatedly extracted with ethyl acetate. The combined organic layer was washed with water and brine, dried over magnesium sulfate, and concentrated. The residue was triturated with ether to afford 0.019 g (40%) of 3-(2-chloro-phenyl)-6-fluoro-2-[2-(2-fluoro-phenyl)-2-hydroxy-vinyl]-3H-quinazolin-4-one as a yellow solid which had: mp 217-219° C; NMR δ 7.85 (dd, J = 2, 8 Hz, 1 H), 7.76 (dt, J = 2, 8 Hz, 1 H), 7.63 (dd, J = 2, 7 Hz, 1 H), 7.54-7.46 (sym m, 2 H), 7.43 (dd, J = 3, 7 Hz, 1 H), 7.42-7.30 (m, 3 H), 7.15 (t, J = 8 Hz, 1 H), 6.95 (dd, J = 8, 11 Hz, 1 H), 5.18 (s, 1 H). Analysis calculated for C₂₂H₁₃CIFN₂O₂: C, 64.32; H, 3.19; N, 6.82. Found: C, 63.97; H, 3.05; N, 6.75.

### Example 5

### 3-(2-Chloro-phenyl)-6-fluoro-2-[2-(2-fluoro-phenyl)-2-hydroxy-vinyl]-3H-quinazolin-4-one

A solution of chromium trioxide (0.072 g, 0.70 mmol) in methylene chloride (2 mL) and pyridine (0.12 mL, 1.4 mmol) was stirred 15 min. 3-(2-Chloro-phenyl)-6-fluoro-2-[2-(2-fluoro-phenyl)-2-hydroxy-ethyl]-3H-quinazolin-4-one (0.051 g, 0.12 mmol, product of example 1) was added and the mixture was allowed to stir overnight at ambient temperature. The reaction was diluted with ethyl acetate and stirred 15 min. The mixture was filtered through a mixture of celite and silica gel and the filter pad was well rinsed with ethyl acetate. The filtrate was dried over magnesium sulfate and concentrated to afford 0.010 g (20 %) of 3-(2-chloro-phenyl)-6-fluoro-2-[2-(2-fluoro-phenyl)-2-hydroxy-vinyl]-3H-quinazolin-4-one as a yellow solid which had NMR identical to that reported in example 4.

### Example 6

### 3-(2-Chloro-phenyl)-6-fluoro-2-[2-(2-fluoro-phenyl)-vinyl]-3H-quinazolin-4-one

A mixture of 3-(2-chloro-phenyl)-6-fluoro-2-[2-(2-fluoro-phenyl)-2-hydroxy-ethyl]-3H-quinazolin-4-one (0.10 g, 0.24 mmol, product of example 1), dioxane (4.5 mL), and trifluoroacetic anhydride (0.5 mL, 3.53 mmol) was stirred ovemight at ambient temperature. The reaction was diluted with water and adjusted to pH 12 by addition of 1 N sodium hydroxide. The mixture was repeatedly extracted with ethyl acetate and the combined organic phase was washed with brine, dried over magnesium sulfate and concentrated. The residue was flash chromatographed on silica gel (9 g) with elution proceeding as follows: 10% ethyl acetate / hexane (100 mL), nil; 20% ethyl acetate / hexane (50 mL), 0.054 g (57%) of 3-(2-chloro-phenyl)-6-fluoro-2-[2-(2-fluoro-phenyl)-vinyl]-3H-quinazolin-4-one as a yellow solid which had: mp 193-195° C; NMR δ 8.06 (d, J = 15.5 Hz, 1 H), 7.92 (dd, J = 3, 8 Hz, 1 H), 7.81 (dd, J = 5, 9 Hz, 1 H), 7.63 (m, 1 H), 7.56-7.46 (m, 3 H), 7.38 (m, 1 H), 7.30-7.22 (m, 2 H), 7.10-6.95 (m, 2 H), 6.42 (d, J = 15.5 Hz, 1 H); ACPI MS m/z = 395.1 (P⁺¹).

### Example 7

### 3-(2-Chloro-phenyl)-2-[2-(6-diethylaminomethyl-pyridin-2-yl)-vinyl]-6-fluoro-3H-quinazolin-4-one Maleate

A mixture of 3-(2-chloro-phenyl)-2-[2-(6-diethylaminomethyl-pyridin-2-yl)-2-hydroxy-ethyl]-6-fluoro-3H-quinazolin-4-one (0.10 g, 0.21 mmol, product of example 2), dioxane (9 mL), and trifluoroacetic anhydride (1 mL, 7.07 mmol) was stirred overnight at ambient temperature. The reaction was diluted with water and adjusted to pH 12 by addition of 1 N sodium hydroxide. The mixture was repeatedly extracted with ethyl acetate and the combined organic phase was washed with brine, dried over magnesium sulfate and concentrated to afford an oily brown solid. This residue was triturated with methylene chloride. Concentration of the methylene chloride solution left an oil which was dissolved in ethyl acetate (1 mL) and treated with maleic acid (0.0235 g, 0.20 mmol, predissolved in 0.6 mL of ethyl acetate with warming). The salt which precipitated upon stirring ovemight was collected and dried under a stream of nitrogen to afford 0.050 g (41%) of 3-(2-chloro-phenyl)-2-[2-(6-diethylaminomethyl-pyridin-2-yl)-vinyl]-6-fluoro-3H-quinazolin-4-one maleate as a tan solid which had: mp 169.5-170.5° C. Analysis calculated for C₂₆H₂₄ClFN₄O · C₄H₄O₄ · 0.25 H₂O: C, 61.75; H, 4.92; N, 9.60. Found: C, 61.70; H, 5.03; N, 9.45.

### Example 8

### 3-(2-Chloro-phenyl)-2-[2-(6-diethylaminomethyl-pyridin-2-yl)-2-hydroxy-vinyl]-6-fluoro-3H-quinazolin-4-one

To a mixture of benzene (1 mL) and dimethyl sulfoxide (1 mL) and 3-(2-chloro-phenyl)-2-[2-(6-diethylaminomethyl-pyridin-2-yl)-2-hydroxy-ethyl]-6-fluoro-3H-quinazolin-4-one (0.050 g, 0.10 mmol, product of example 2) was added ethyl 2-dimethylaminoethyl carbodiimide (0.059 g, 0.31 mmol). After two minutes phenylphosphonic acid (0.008 g) was added all at once. After stirring 3 h, additional ethyl 2-dimethylaminoethyl carbodiimide (0.060 g, 0.31 mmol) was added and the mixture was stirred at ambient temperature overnight. Additional ethyl 2-dimethylaminoethyl carbodiimide (0.090 g, 0.46 mmol) was added and the mixture stirring was continued at ambient temperature 72 h longer. The reaction was diluted with water and repeatedly extracted with ethyl acetate. The combined organic layer was washed with water and brine, dried over magnesium sulfate, and concentrated to a yellow oil. The residue was triturated with ether to afford 0.007 g (14%) of 3-(2-chloro-phenyl)-2-[2-(6-diethylaminomethyl-pyridin-2-yl)-2-hydroxy-vinyl]-6-fluoro-3H-quinazolin-4-one as an amorphous light yellow solid which had: NMR δ 7.86-7.82 (m, 2 H), 7.69 (t, J = 8.5 Hz, 1 H), 7.64-7.60 (m, 1H), 7.54-7.47 (sym m, 2 H), 7.47-7.36 (m, 4 H), 5.99 (s, 1 H), 3.56 (s, 2 H), 2.43 (q, J = 7 Hz, 4 H), 0.96 (t, J = 7 Hz, 6 H); ACPI MS m/z = 479.1 (P⁺¹).

### Example 9

### 3-(2-Chloro-phenyl)-6-fluoro-2-[2-(6-pyrrolidin-1-ylmethyl-pyridin-2-yl)-vinyl]-3H-quinazolin-4-one

A mixture of 3-(2-chloro-phenyl)-2-[2-(6-pyrrolidin-1-ylmethyl-pyridin-2-yl)-2-hydroxy-ethyl]-6-fluoro-3H-quinazolin-4-one (0.130 g, 0.27 mmol, product of example 3), dioxane (9 mL), and trifluoroacetic anhydride (1 mL, 7.07 mmol) was stirred 72 h at ambient temperature. The reaction was diluted with water and adjusted to pH 11 by addition of 1 N sodium hydroxide. The mixture was repeatedly extracted with ethyl acetate and the combined organic phase was washed with brine, dried over magnesium sulfate and concentrated to afford an oil. This residue was flash chromatographed on silica gel (20 x 100 mm) collecting 5 mL fractions. Elution proceeded as follows: 4% methanol / 0.4% ammonium hydroxide / methylene chloride (110 mL), nil; 4% methanol / 0.4% ammonium hydroxide / methylene chloride (55 mL), first pure product and then a mixture of product and starting material. The pure fractions were combined and concentrated to afford 0.015 g (11%) of 3-(2-Chloro-phenyl)-6-fluoro-2-[2-(6-pyrrolidin-1-ylmethyl-pyridin-2-yl)-vinyl]-3H-quinazolin-4-one as a pale oil which solidified on standing and had: mp 191-194° C; NMR δ 7.94 (d, J = 15 Hz, 1 H), 7.91 (dd, J = 3, 9 Hz, 1 H), 7.78 (dd, J = 5, 9 Hz, 1 H), 7.62-7.44 (m, 5 H), 7.40-7.38 (m, 1 H), 7.24 (d, J = 8 Hz, 1 H), 7.14 (d, J = 8 Hz, 1 H), 6.85 (d, J = 15 Hz, 1 H), 3.63 (s, 2 H), 2.49 (br s, 4 H), 1.74 (br s, 4 H). Analysis calculated for C₂₆H₂₂ClFN₄O · 0.5 H₂O: C, 66.45; H, 4.93; N, 11.92. Found: C, 66.29; H, 4.97; N, 11.62.

### Example 10

### (+) 3-(2-Chloro-phenyl)-2-[2-(6-diethylaminomethyl-pyridin-2-yl)-vinyl]-6-fluoro-3H-quinazolin-4-one Mesylate

3-(2-Chloro-phenyl)-2-[2-(6-diethylaminomethyl-pyridin-2-yl)-2-hydroxy-ethyl]-6-fluoro-3H-quinazolin-4-one was prepared following the procedure of Example 2 as a mixture of diastereomers (each as a single enantiomer) commencing with (+) 3-(2-chloro-phenyl)-6-fluoro-2methyl-3H-quinazolin-4-one.

The diastereomeric mixture of 3-(2-chloro-phenyl)-2-[2-(6-diethylaminomethyl-pyridin-2-yl)-2-hydroxy-ethyl]-6-fluoro-3H-quinazolin-4-one (0.0901 g, 0.19 mmol) was dissolved in dimethoxyethane (9 mL), and trifluoroacetic anhydride (1 mL, 7.07 mmol) was added. The reaction was stirred 36 h at ambient temperature. The mixture was poured onto ice and the pH was adjusted tp 12 by addition of 1N sodium hydroxide. The mixture was repeatedly extracted with ethyl acetate and the combined organic pahse was washed with brine, dried over magnesium sulfate and concentrated to afford an oil which slowly solidified (0.0832 g). This solid was dissolved in ethyl acetate (1 mL) and treated with 1 N methanesulfonic acid in ethyl acetate (0.179 mL, 0.179 mmol). The salt which precipitated upon stirring overnight was collected and dried under a stream of nitrogen to afford 0.047 g (44.9% for the dehydration and salt formation steps) of (+) 3-(2-chloro-phenyl)-2-[2-(6-diethylaminomethyl-pyridin-2-yl)-vinyl]-6-fluoro-3H-quianzolin-4-one mesylate as a white solid which had: mp 185-186°C; [α]_{D} = +37.2° c=0.436 (methanol). Analysis calculated for C₂₆H₂₄ClFN₄O · 1.5 CH₄SO₃: C, 54.50; H, 4.82; N, 9.24. Found: C, 54.83; H, 4.93; N, 9.25. HPLC retention time=10.384 min [4.6 mm x 25 cm chiralcel OD column, 1 mL/min flow rate, UV detection (250nM)].

### Example 11

### (-) 3-(2-Chloro-phenyl)-2-[2-(6-diethylaminomethyl-pyridin-2-yl)-vinyl]-6-fluoro-3H-quinazolin-4-one Mesylate

3-(2-Chloro-phenyl)-2-[2-(6-diethylaminomethyl-pyridin-2-yl)-2-hydroxy-ethyl]-6-fluoro-3H-quinazolin-4-one was prepared following the procedure of Example 2 as a mixture of diastereomers (each as a single enantiomer) commencing with (-) 3-(2-chloro-phenyl)-6-fluoro-2-methyl-3H-quinazolin-4-one.

The diastereomeric mixture of 3-(2-chloro-phenyl)-2-[2-(6-diethylaminomethyl-pyridin-2-yl)-2-hydroxy-ethyl]-6-fluoro-3H-quinzolin-4-one was converted to (-) 3-(2-Chloro-phenyl)-2-[2-(6-diethylaminomethyl-pyridin-2-yl)-vinyl]-6-fluoro-3H-quinazolin-4-one mesylate following the procedure of Example 10 (36.4% for the final dehydration and salt formation steps) and had: mp 185-186°C; [α]_{D} = -36.33° c=0.40 (methanol). Analysis calculated for C₂₆H₂₄ClFN₄O · 1.5 CH₄SO₃ ^{.} 0.5 H₂O: C, 52.09; H, 5.26; N, 8.52. Found: C, 52.10; H, 5.26; N, 8.52. HPLC retention time=12.785 min [4.6 mm x 25 cm chiralcel OD column, 1mL/min flow rate, UV detection (250nM)].

### Example 12

### Preparative HPLC separation of (+) 3-(Chloro-phenyl)-6-fluoro-2-methyl-3H-quinazolin-4-one and (-) 3-(2-Chloro-phenyl)-6-fluoro-2-methyl-3H-quinazolin-4-one

Racemic 3-(2-chloro-phenyl)-6-fluoro-2-methyl-3H-quinazolin-4-one (1.0 g) was dissolved in 50 mL of 0.1% diethylamine/isopropanol (final concentration 20mg/mL) and applied to a preparative HPLC column (5 x 50cm chiralcel AD) and eluted with 90/10/0.1 heptane/isopropanol/diethylamine at a flow rate of 95 mL/min. The eluent was monitored with ultraviolet detection at 250 nM. Two fractions were collected, the first component centered around an elution time of 13.5 min and the second around an elution time of 22 min. The eluent from three cycles with elution time of 13.5 min were combined and concentrated to give a light yellow solid. The solid was triturated with hexane to afford 1.217 g of (+) 3-(2-chloro-phenyl)-6-fluoro-2-methyl-3H-quinazolin-4-one as an off-white powder. The product was further purified by flash chromatography on silica gel eluting with 30-40% ethyl acatete/hexanes. Product containing fractions [tlc R_{f} = 0.37(on silica gel developed with 50% ethyl acetate/hexanes)] were combined and concentrated to give the off-white product which had NMR (CDCl₃) δ 7.89 (dd, J = 3, 8.5 Hz, 1H), 7.69 (dd, J = 5, 9 Hz, 1H), 7.63-7.58 (m, 1H), 7.51-7.44 (m, 3H), 7.35-7.30 (m, 1H), 2.20 (s, 3H); [α]_{D} = +90.1° (c=0.555 in chloroform).

The eluent from the same three cycles with elution time of 22 min were concentrated and triturated with hexanes in the same fashion to afford 1.24 g of (-)3-(2-chloro-phenyl)-6-fluoro-2-methyl-3H-quinazolin-4-one as an off-white solid which had: [α]_{D} = -90.7° (c=0.89 in chloroform).

All other physical characteristics were identical to the atropisomer.

### Example 13

### Preparative HPLC separation of (+) 3-(2-Methylpyrid-3-yl)-6-fluoro-2-methyl-3H-quinazolin-4-one and (-) 3-(2-Methylpyrid-3-yl)-6-fluoro-2-methyl-3H-quinazolin-4-one

Racemic 3-(2-methylpyrid-3-yl)-6-fluoro-2-methyl-3H-quinazolin-4-one (2.0tg) was dissolved in 100 mL of 0.1% diethylaminelisopropanol (final concentration 20 mg/mL) and applied to a preparative HPLC column (5 x 50 cm Chiralcel AD) and eluted with 90/10/0.1 heptane/isopropanol/ diethylamine at a flow rate of 95 ml/min. The eluent was monitored with ultraviolet detection at 265 nM. Two fractions were collected, the first component centered around an elution time of 12.7 min and the second around an elution time of 16.6 min. The eluent from four cycles with elution time of 12.7 min were combined and concentrated to give a light yellow solid. The solid was triturated with 5% ether/hexane to afford 0.812 g of (+)-3-(2-methylpyrid-3-yl)-6-fluoro-2-methyl-3H-quinazolin-4-one as an off white powder which had: mp 128-129°C; [α]_{D} = + 60.4° (c = 0.505 in chloroform).

The eluent from the same four cycles with elution time of 16.6 min were concentrated and triturated with 5% ether/hexane in the same fashion to afford 0.749 g of (-)-3-(2-methylpyrid-3-yl)-6-fluoro-2-methyl-3H-quinazolin-4-one as an off white solid which had: mp 129-130°C; [α]_{D} = -57.48° (c = 0.515 in chloroform).

## Claims

1. A process for preparing a compound of the formula 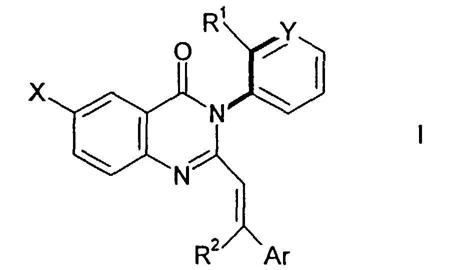 and the pharmaceutically acceptable salts thereof, wherein
R¹ is halo, cyano, (C₁-C₆)alkyl, perfluoro(C₁-C₆)alkyl or -COO(C₁-C₆)alkyl;
R² is hydrogen or hydroxy;
X is hydrogen, hydroxy, halo, trifluoromethyl, nitro, (C₁-C₆)alkyl optionally substituted by (C₁-C₆)alkoxy, hydroxy, amino (C₁-C₆)alkylamino or ((C₁-C₆)alkyl)₂amino; (C₁-C₇)cydoalkyl, (C₁-C₆)alkoxy, (C₁-C₆)acyl, (C₁-C₆)alkyl-CO₂- (C₁-C₆)alkyl, (C₁-C₆)alkyl-OCO-(C₁-C₆)alkyl, (C₁-C₆)alkyl-CO₂-, NCO, R³OCO-, R³NHCO- wherein R³ is hydrogen or (C₁-C₆)alkyl; ((C₁-C₆)alkyl)amino-CO- or phenyl optionally substituted by halo, (C₁-C₆)alkyl, cyano or trifluoromethyl;
Y is nitrogen or CH;
Ar is a group of the formula 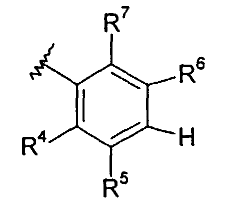 wherein R⁴ and R⁷ are each independently selected from hydrogen, halo, trifluoromethyl, (C₁-C₆)alkoxy optionally substituted with one to three halogen atoms; (C₁-C₆)alkylthio, hydroxy, (C₁-C₆)acyl, cyano, (C₁-C₆)alkyl optionally substituted by amino, (C₁-C₆)alkylamino, ((C₁-C₆)alkyl)₂amino, (C₃-C₇)cycloalkylamino, hydroxy, (C₁-C₆)alkoxy, HCO- H₂NCO-, (C₁-C₆)alkylamino-CO-, ((C₁-C₆)alkyl)₂amino-CO-, (C₃-C₇)cycloalkylamino-CO-, HCO-NH-, (C₁-C₆)alkyl-CO-NH-, HCO-N((C₁-C₆)alkyl)-, (C₁-C₆)alkyl-CO-N((C₁-C₆)alkyl)-, one to three halogen atoms, R⁸O- or R⁸O-CO- wherein R⁸ is hydrogen, (C₁-C₆)alkyl, (C₁-C₆)acyl, (C₁-C₆)alkoxy-CO-, (C₁-C₆)alkylamino-CO-, or ((C₁-C₆)alkyl)₂amino-CO-;
R⁵ and R⁶ are each independently selected from hydrogen, halo, CF₃, (C₁-C₆)alkyl optionally substituted with one to three halogen atoms, (C₁-C₆)alkoxy optionally substituted with one to three halogen atoms, (C₁-C₆)alkylthiol, amino-(CH₂)ₚ-, (C₁-C₆)alkyl-NH-(CH₂)ₚ-, di(C₁-C₆)alkyl-N-(CH₂)ₚ-, (C₃-C₇)cycloalkyl-NH-(CH₂)ₚ-, amino-(C₁-C₆)alkyl-NH-(CH₂)ₚ-, (C₁-C₆)alkyl-NH-(C₁-C₆)alkyl-NH-(CH₂)ₚ-, di(C₁-C₆)alkyl-N((C₁-C₆)alkyl)-(C₁-C₆)alkyl-NH-(CH₂)ₚ-, di(C₁-C₆)alkyl-N-(C₁-C₆)alkyl-N-(CH₂)ₚ-, H₂N-(C=O)-(CH₂)ₚ-, (C₁-C₆)alkyl-HN-(C=O)-(CH₂)ₚ-, di(C₁-C₆)alkyl-N-C=O)-(CH₂)ₚ, (C₃-C₇)cycloalkyl-NH-(C=O)-(CH₂)ₚ-, R¹³O-(CH₂)ₚ-, R¹³O-(C=O)-(CH₂)ₚ-, H(O=C)-O-, H(O=C)-O-(C₁-C₆)alkyl-, H(O=C)-NH-(CH₂)ₚ-, (C₁-C₆)alkyl-(O=C)-NH-(CH₂)ₚ-, -CHO, H-(C=O)-(CH₂)ₚ-, (C₁-C₆)alkyl-(C=O)-(CH₂)ₚ-, (C₁-C₆)alkyl-(O=C)-N((C₁-C₆)alkyl)-(CH₂)ₚ-, H(O=C)-N((C₁-C₆)alkyl)-(CH₂)ₚ-, HO-(C₁-C₆)alkyl-N((C₁-C₆)alkyl)-(CH₂)ₚ-, (C₁-C₆)alky-(C=O)-O-NH-(CH₂)ₚ-, amino-(C₁-C₆)alkyl-(C=O)-O-(CH₂)ₚ-, (C₁-C₆)alkyl-NH-(C₁-C₆)alkyl-(C=O)-O-(CH₂)ₚ-, di(C₁-C₆)alkyl-N-C₁-C₆)alkyl-(C=O)-O-(CH₂)ₚ-, amino-(C₁-C₆)alkyl-O-(C=O)-(CH₂)ₚ-, (C₁-C₆)alkyl-NH-(C₁-C₆)alkyl-O-(C=O)-(CH₂)ₚ-, di(C₁-C₆)alkyl-N-(C₁-C₆)alkyl-O-(C=O)-(CH₂)ₚ-, hydroxy, hydroxy-(C₁-C₆)alkyl-, hydroxy-(C₁-C₆)alkyl-NH-(CH₂)ₚ-, (C₁-C₆)alkyl-O-(C₁-C₆)alkyl-, -CN, piperidine-(CH₂)ₚ-_{,} pyrrolidine-(CH₂)ₚ-, and 3-pyrroline-(CH₂)ₚ-, wherein said piperidine, pyrrolidine and 3-pyrroline of said piperidine-(CH₂)ₚ-, pyrrolidine-(CH₂)ₚ- and 3-pyrroline-(CH₂)ₚ- moieties may optionally be substituted on any of the ring carbon atoms capable of supporting and additional bond, with a substituent independently selected from halo, CF₃, (C₁-C₆)alkyl optionally substituted with one to three halogen atoms, (C₁-C₆)alkoxy optionally substituted with one to three halogen atoms, (C₁-C₆)alkylthiol, amino-(CH₂)ₚ-, (C₁-C₆)alkyl-NH-(CH₂)ₚ-, di(C₁-C₆)alkyl-N-(CH₂)ₚ-, (C₃-C₇)cycloalkyl-NH-(CH₂)ₚ-, amino-(C₁-C₆)alkyl-NH-(CH₂)ₚ-, (C₁-C₆)alkyl-NH-(C₁-C₆)alkyl-NH-(CH₂)ₚ-, di(C₁-C₆)alkyl-N-(C₁-C₆)alkyl-NH-(CH₂)ₚ-, (C₁-C₆)alkyl-O-(C₁-C₆)alkyl-, di(C₁-C₆)alkyl-N-(C₁-C₆)alkyl-N((C₁-C₆)alkyl)-(CH₂)ₚ-, H₂N-(C=O)-(CH₂)ₚ-, (C₁-C₆)alkyl-HN-(C=O)-(CH₂)ₚ-, di(C₁-C₆)alkyl-N-(C=O)-(CH₂)ₚ, (C₃-C₇)cycloalkyl-NH-(C=O)-(CH₂)ₚ-, R¹³O-(CH₂)ₚ-, R¹³O-(C=O)-(CH₂)ₚ-, H(O=C)-O-, H(O=C)-O-(C₁-C₆)alkyl-, H(O=C)-NH-(CH₂)ₚ-, (C₁-C₆)alkyl-(O=C)-NH-(CH₂)ₚ-, -CHO, H-(C=O)-(CH₂)ₚ-, (C₁-C₆)alkyl-(C=O)-, (C₁-C₆)alkyl-(O=C)-N((C₁-C₆)alkyl)-(CH₂)ₚ-, H(O=C)-N((C₁-C₆)alkyl)-(CH₂)ₚ-, HO-(C₁-C₆)alkyl-N((C₁-C₆)alkyl)-(CH₂)ₚ-, (C₁-C₆)alkyl- (C=O)-O-NH-(CH₂)ₚ-, amino-(C₁-C₆)alkyl-(C=O)-O-(CH₂)ₚ-, (C₁-C₆)alkyl-NH-(C₁-C₆)alkyl-(C=O)-O-(CH₂)ₚ-, di(C₁-C₆)alkyl-N-(C₁-C₆)alkyl-(C=O)-O-(CH₂)ₚ-, hydroxy, hydroxy-(C₁-C₆)alkyl-, hydroxy-(C₁-C₆)alkyl-NH-(CH₂)ₚ-, and -CN;
p is 0 to 6;
or Ar is a group of the formula 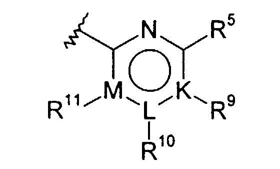 wherein K, L and M are each independently selected from carbon or nitrogen;
R⁵ is defined as above;
R⁹ and R¹⁰ are each independently selected from hydrogen, cyano, (C₁-C₆)alkyl, halo, trifluoromethyl, HCO- or (C₁-C₆)alkoxy;
R¹¹ is selected from hydrogen, cyano, halo, trifluoromethyl, (C₁-C₆)alkoxy, HCO-, (C₁-C₆)alkyl, optionally substituted by amino, (C₁-C₆)alkylamino or ((C₁-C₆)alkyl)₂amino;
or Ar is a group of the formula 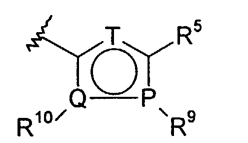 wherein T is CH, N, NH, O or S;
P and Q are each independently selected from carbon, nitrogen, oxygen or sulfur;
R⁵, R⁹ and R¹⁰ are defined as above;
with the proviso that only one of K, L or M can be nitrogen and when K, L or M is nitrogen then its respective R⁹, R¹⁰ or R¹¹ is absent; and
with the proviso that only one of P, Q or T can be oxygen or sulfur and at least one of P, Q or T must be a heteroatom;
comprising either (a) dehydrating the quinazolin-4-one derivative of the formula 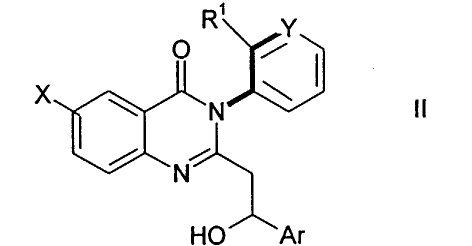 with a dehydration agent to form the compound of formula **I**, wherein R¹, X, Y and Ar are defined as above and R² is hydrogen; or
(b) oxidizing the compound of formula **II** with an oxidizing agent to form the compound of formula I, wherein R¹, X, Y and Ar are defined as above and R² is hydroxy.

2. A process according to claim 1, wherein the dehydration agent is selected from acetic anhydride, trifluoroacetic anhydride, acetyl chloride, methanesulfonyl chloride, Burgess salt, thienyl chloride, phosphorus triichloride or phosphorus oxychloride.

3. A process according to claim 1, wherein the oxidizing agent is selected from chromium trioxide, pyridinium dichromate, pyridinium chlorochromate, dicyclohexylcarbodiimide or ethyl 2-dimethylaminoethyl carbodiimide.

4. A process according to claim 1, wherein the compound of the formula 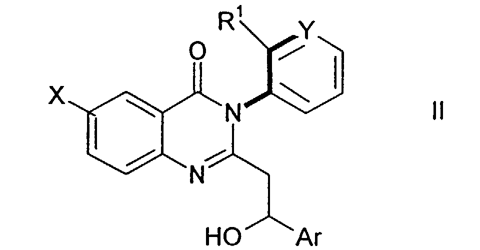 is formed by (a) deprotonating the methyl group at the 2-position of a quinazolin-4-one compound having the formula 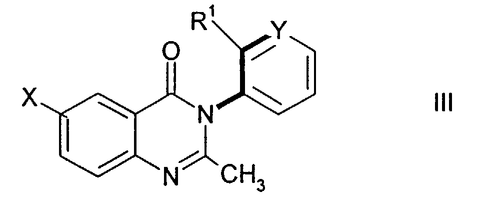 wherein X, Y and R¹ are as defined above, with a base to form the corresponding anion; and (b) reacting the anionic intermediate so formed with an aromatic aldehyde of the formula Ar-CHO, wherein Ar is defined as above, to form the compound of the formula **II**.

5. A process according to claim 4, wherein the base is selected from lithium diisopropylamide, lithium hexamethyldisilylamide, sodium hexamethyldisilylamide, potassium hexamethyldisilylamide or lithium 2,2,6,6-tetramethylpiperidide.

6. A process according to claim 1 for preparing a compound of the formula I from formula II wherein the compound of formula II is prepared by the steps of:
(a) deprotonating the methyl group at the 2-position of a quinazolin-4-one compound having the formula 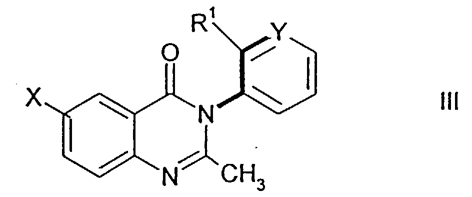 wherein X, Y and R¹ are as defined in claim 1 with a base to form the corresponding anion; and
(b) reacting the anionic intermediate so formed of Step (a) with an aromatic aldehyde, Ar-CHO, to form a quinazolin-4-one derivative of the formula 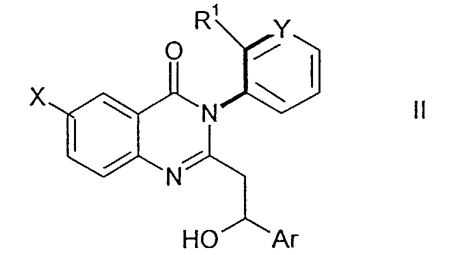 wherein R¹, X, Y and Ar are defined in claim 1;followed by either:
(c) dehydrating the product so formed of Step (b) with a dehydration agent to form a quinazolin-4-one derivative of the formula 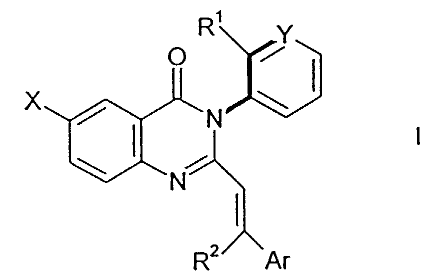 wherein R¹, X, Y and Ar are as defined in claim 1 and R² is hydrogen; or
(d) oxidizing the product so formed of Step (b) with an oxidizing agent to form a quinazolin-4-one derivative of the formula 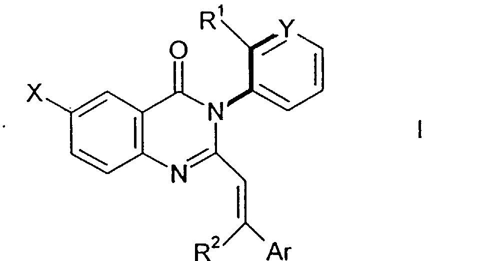 wherein R¹, X, Y and Ar are as defined in claim 1 and R² is hydroxy.

7. A process for preparing a compound of the formula 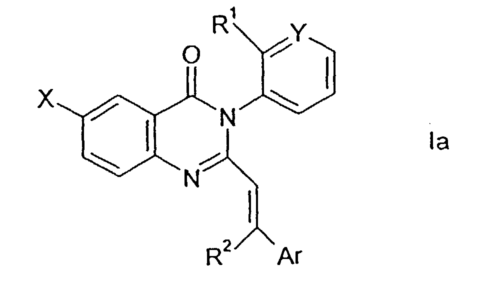 and the pharmaceuticall acceptable salts thereof, wherein R¹, R², X and Ar are as defined in claim 1 comprising either (a) dehydrating the quinazolin-4-one derivative of the formula 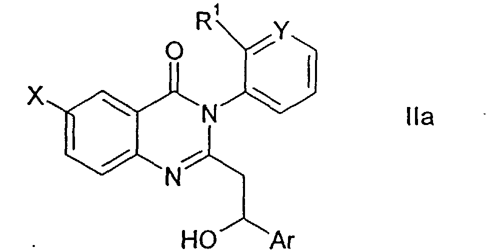 with a dehydration agent to form the compound of formula **Ia**, wherein R¹, X, Y and Ar are defined as above and R² is hydrogen; or
(b) oxidizing the compound of formula **IIa** with an oxidizing agent to form the compound of formula la, wherein R¹, X, Y and Ar are defined as above and R² is hydroxy.

8. A process according to claim 7, wherein the dehydration agent is selected from acetic anhydride, trifluoroacetic anhydride, acetyl chloride, methanesulfonyl chloride, Burgess salt, thienyl chloride, phosphorus triichloride or phosphorus oxychloride.

9. A process according to claim 7, wherein the oxidizing agent is selected from chromium trioxide, pyridinium dichromate, pyridinium chlorochromate, dicyclohexylcarbodiimide or ethyl 2-dimethylaminoethyl carbodiimide.

10. A process according to claim 1, wherein the compound of the formula 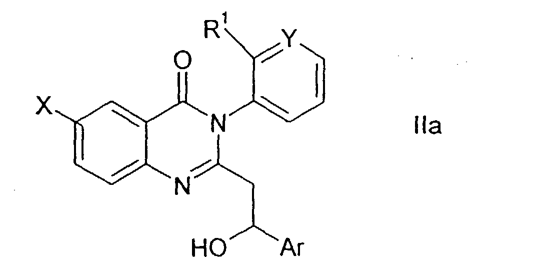 is formed by (a) deprotonating the methyl group at the 2-position of a quinazolin-4-one compound having the formula 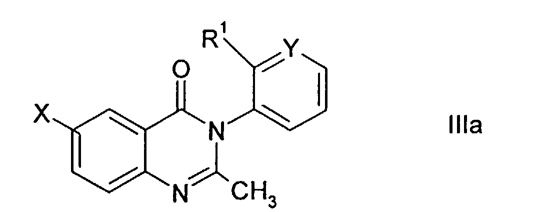 wherein X, Y and R¹ are as defined above, with a base to form the corresponding anion; and (b) reacting the anionic intermediate so formed with an aromatic aldehyde of the formula Ar-CHO, wherein Ar is defined as above, to form the compound of the formula **IIa**.

11. A process according to claim 10, wherein the base is selected from lithium diisopropylamide, lithium hexamethyldisilylamide, sodium hexamethyldisilylamide, potassium hexamethyldisilylamide or lithium 2,2,6,6-tetramethylpiperidide.

12. A process for preparing a compound of the formula 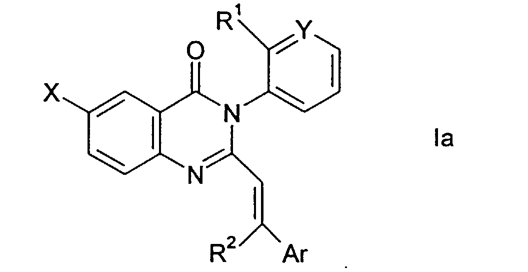 and the pharmaceutically acceptable salts thereof, wherein R¹, R², X and Ar are as defined in claim 1 which process comprises the steps of:
(a) deprotonating the methyl group at the 2-position of a quinazolin-4-one compound having the formula 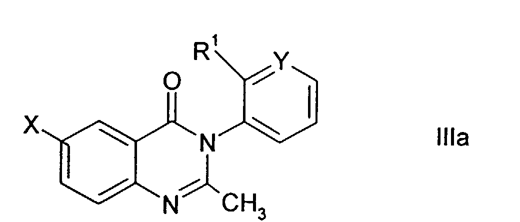 wherein X, Y and R¹ are as defined above, with a base to form the corresponding anion;
b) reacting the anionic intermidiate so formed of Step (a) with an aromatic aldehyde, Ar-CHO, to form a quinazolin-4-one derivative of the formula 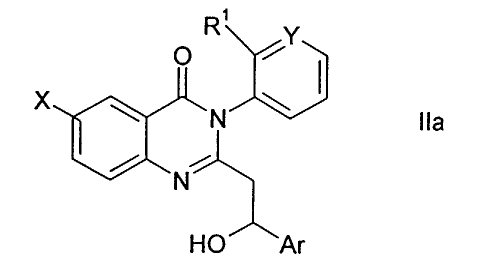 wherein R¹, X, Y and Ar are defined as above; followed by either:
c) dehydrating the product so formed of Step (b) with a dehydrating agnet to form a quinazolin-4-one derivative of the formula 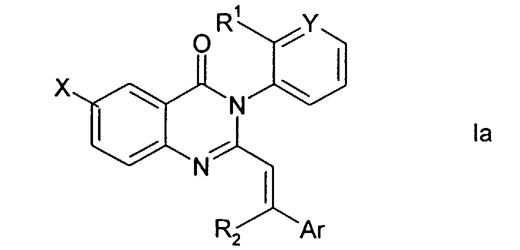 wherein R¹, X, Y and Ar are defined as above and R² is hydrogen; or
d) oxidizing the product so formed of Step (b) with an oxidizing agent to form a quinazolin-4-one derivative of the formula 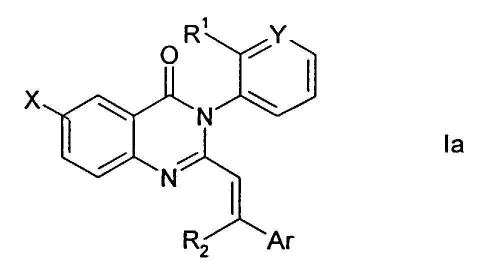 wherein R¹, X, Y and Ar are as defined as above and R² is hydroxy.

13. A compound of the formula 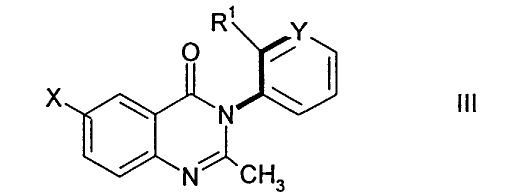 wherein R¹ is halo, cyano, (C₁-C₆)alkyl, perfluoro(C₁-C₆)alkyl or -COO(C₁-C₆)alkyl;
X is hydrogen, hydroxy, halo, trifluoromethyl, nitro, (C₁-C₆)alkyl optionally substituted by (C₁-C₆)alkoxy, hydroxy, amino (C₁-C₆)alkylamino or ((C₁-C₆)alkyl)₂amino; (C₃-C₇)cycloalkyl, (C₁-C₆)alkoxy, (C₁-C₆)acyl, (C₁-C₆)alkyl-CO₂-(C₁-C₆)alkyl, (C₁-C₆)alkyl-OCO-(C₁-C₆)alkyl, (C₁-C₆)alkyl-CO₂-, NCO, R³OCO-, R³NHCO- wherein R³ is hydrogen or (C₁-C₆)alkyl; ((C₁-C₆)alkyl)amino-CO- or phenyl optionally substituted by halo, (C₁-C₆)alkyl, cyano or trifluoromethyl; and
Y is nitrogen or CH.

14. A compound of the formula 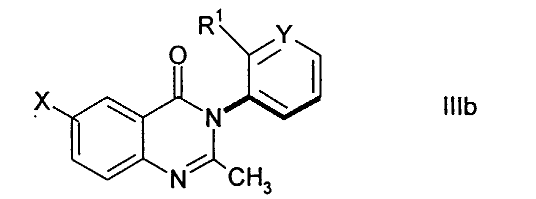 wherein R¹ is halo, cyano, (C₁-C₆)alkyl, perfluoro(C₁-C₆)alkyl or -COO(C₁-C₆)alkyl;
X is hydrogen, hydroxy, halo, trifluoromethyl, nitro, (C₁-C₆)alkyl optionally substituted by (C₁-C₆)alkoxy, hydroxy, amino (C₁-C₆)alkylamino or ((C₁-C₆)alkyl)₂amino; (C₃-C₇)cycloalkyl, (C₁-C₆)alkoxy, (C₁-C₆)acyl, (C₁-C₆)alkyl-CO₂- (C₁-C₆)alkyl, (C₁-C₆)alkyl-OCO-(C₁-C₆)alkyl, (C₁-C₆)alkyl-CO₂-, NCO, R³OCO-, R³NHCO- wherein R³ is hydrogen or (C₁-C₆)alkyl; ((C₁-C₆)alkyl)amino-CO- or phenyl optionally substituted by halo, (C₁-C₆)alkyl, cyano or trifluoromethyl; and
Y is nitrogen or CH.
